# EUROPEAN PATENT SPECIFICATION

(11) **EP 3 741 400 B1**
(45) Date of publication and mention of the grant of the patent: **11.03.2026**
(21) Application number: 18899891.8
(22) Date of filing: 15.01.2018
(51) Int. Cl.: A61L 31/12, A61L 31/04, B32B 5/32, B32B 27/00, B32B 27/26, A61L 31/14

(54) **ANTI-ADHESION COMPOSITION**
ANTIHAFTZUSAMMENSETZUNG
COMPOSITION ANTIADHÉSIVE

(43) Date of publication of application: 25.11.2020
(73) Proprietor: Mochida Pharmaceutical Co., Ltd., Tokyo 160-8515 (JP); The University of Tokyo, Bunkyo-ku, Tokyo 113-8654 (JP)
(72) Inventor: ITO, Taichi, Tokyo 113-8654 (JP); OHTA, Seiichi, Tokyo 113-8654 (JP); HASEGAWA, Kiyoshi, Tokyo 113-8654 (JP); ISAJI, Mitsuko, Tokyo 160-8515 (JP); SHIMIZU, Satoshi, Tokyo 160-8515 (JP); TANAKA, Daichi, Tokyo 160-8515 (JP)
(74) Representative: dompatent
(86) International application number: PCT/JP2018/000874
(87) International publication number: WO 2019/138583

(56) References cited:
- EP-A1- 1 135 418
- EP-A1- 3 485 921
- WO-A1-2016/114355
- WO-A1-2017/159700
- WO-A1-2018/012605
- WO-A1-93/13136
- JP-A- 2002 530 440
- JP-A- 2003 062 063
- JP-A- 2003 509 468
- JP-A- 2007 075 425
- JP-A- 2016 502 874
- US-A1- 2012 039 959
- US-A1- 2012 039 959
- HYUN JOON KONG ET AL: "Controlling Rigidity and Degradation of Alginate Hydrogels via Molecular Weight Distribution", BIOMACROMOLECULES, vol. 5, no. 5, 1 September 2004 (2004-09-01), US, pages 1720 - 1727, XP055663386, ISSN: 1525-7797, DOI: 10.1021/bm049879r
- JIRO NAMBA ET AL: "Modulation of peritendinous adhesion formation by alginate solution in a rabbit flexor tendon model", JOURNAL OF BIOMEDICAL MATERIALS RESEARCH PART B: APPLIED BIOMATERIALS, vol. 80B, no. 1, 9 June 2006 (2006-06-09), US, pages 273 - 279, XP055602314, ISSN: 1552-4973, DOI: 10.1002/jbm.b.30594
- NAMBA J. ET AL.: "Modulation of peritendinous adhesion formation by alginate solution in a rabbit flexor tendon model.", JOURNAL OF BIOMEDICAL MATERIALS RESEARCH. PART B, APPLIED BIOMATERIALS, vol. 80, no. 1, 2007, pages 273 - 279, XP055602314, ISSN: 1552-4973, doi:10.1002/jbm.b.30594
- CHATURVEDI, A. A. ET AL.: "Prevention of postsurgical adhesions using an ultrapure alginate-based gel.", BRITISH JOURNAL OF SURGERY, vol. 100, no. 7, 2013, pages 904 - 910, XP055296836, ISSN: 0007-1323, doi:10.1002/bjs.9131

## Description

### FIELD OF THE INVENTION

The present invention relates to an anti-adhesion material, a method for producing the same and a sponge-like laminate.

### BACKGROUND ART

Adhesions refer to a state where surfaces of tissues that should be separated from each other are connected or fused to each other via fibrous tissue. Adhesions occur in association with injury or inflammation upon which an exudate containing fibrin is emitted on the surface of the tissue, where this exudate is organized such that the tissue surfaces are connected or fused. Adhesions are caused by an injury generated on a surface of a tissue upon a surgical operation, inflammation caused by an injury, and inflammation caused by drying of a tissue surface upon a surgical operation.

Adhesions sometimes cause infertility, bowel passing disorder and chronic pelvic pain. Moreover, in order to separate adhesions that were generated after a surgical operation, another surgical operation may be required. For example, while repeated operations are effective for recurrence of liver cancer, judgement of the propriety of repeated surgery, risks of the treatment, an amount of bleeding upon the operation, operation time and the like are all largely dependent on the prevention of adhesions following the previous operation. Accordingly, there is a need for preventing adhesions and thus various means have been adopted to date for preventing adhesions.

Some of such means for preventing adhesions involve providing a physical barrier between an injury or an inflammation site and the adjacent tissue to prevent the tissues from connecting or fusing with each other. A sheet-like barrier is known as such a physical barrier.

Specifically, examples of such a sheet-like barrier include a polytetrafluoroethylene (PTFE) film (Preclude (trade name) (WL Gore and Associates, Inc.)), a sheet containing hyaluronic acid (HA) and carboxymethyl cellulose (CMC) (Seprafilm (trade name) (Genzyme GmbH)), and an oxidized regenerated cellulose sheet (INTERCEED (trade name) (Johnson & Johnson)). Since the PTFE film among them is not biodegradable, it has a problem of remaining in the body. On the other hand, the sheet containing HA and CMC as well as the oxidized regenerated cellulose sheet are biodegradable but they are unable to completely prevent serious adhesions such as an adhesion caused after hepatic resection, and thus they require further improvement to be effective in adhesion prevention.

Meanwhile, it is known to make a biocompatible material selected from proteins such as collagen or polysaccharides such as carboxymethyl cellulose, hyaluronic acid or alginic acid into a sheet or particles so that it can be used as a medical absorbing material, a medical patch, an anti-adhesion material, a biological tissue reinforcement material or the like (Patent documents 1-6).

Patent Document 7 discloses a bi-layered alginate sheet used as adhesion prevention material. Said material may be prepared by generating a layer of alginate and cross-linking it by sprinking Ca lactate; drying the film; pouring the solution of alginate used to prepare the first layer on the dried film and sprinkling a solution of Ca lactate to promote cross-linking and drying the composite material. The amount of Ca lactate sprinkled on the alginate in the two steps is different and thus the two layers have a different degree of crosslinking and thus a different dissolution rate. The higher the crosslink density , the slower the dissolution rate of the layer. Patent Document 7 also teaches that having a multilayered material wherein one layer dissolves rapidly and the second layer slowly is beneficial for healing in the repair site.

### PRIOR ART DOCUMENTS

### Patent Documents

Patent Document 1: Japanese Unexamined Patent Application Publication No. Showa 48-79870
Patent Document 2: Japanese Unexamined Patent Application Publication No. 2003-126235
Patent Document 3: International Patent Application Publication No. WO 2005/26214
Patent Document 4: Japanese Unexamined Patent Application Publication No. 2011-25013
Patent Document 5: Japanese Unexamined Patent Application Publication No. 2013-165884
Patent Document 6: Japanese Unexamined Patent Application Publication (Translation of PCT) No. 2016-502874
Patent Document 7: US 2012039959

### SUMMARY OF INVENTION

### Problem to be Solved by Invention

Under such circumstances, there has been a need for an anti-adhesion material that has at least one of the following performances: highly effective in preventing adhesions; capable of suppressing both adhesion of a wound and *de novo* adhesion; has no adverse effect on the living body applied; does not interfere with healing of a wound; can also be used for intestinal anastomosis or the like; allows easy application via a trocar upon an endoscopic surgery; capable of being reapplied to adjust the applied position; and the like.

### Means for Solving Problem

The present inventors have gone through intensive studies on an anti-adhesion material that has both advantages of a film (sheet)-like anti-adhesion material and a spray (liquid/gel)-type anti-adhesion material in animal adhesion models assuming various clinical operations. As a result, they found that a biocompatible sponge-like anti-adhesion material comprising a first layer and a second layer with different dissolution rates, specifically, an anti-adhesion material comprising a biocompatible sponge-like laminate that includes a first sponge-like layer containing a low-endotoxin monovalent metal salt of alginic acid with a relatively high weight-average molecular weight and a second sponge-like layer containing a low-endotoxin monovalent metal salt of alginic acid with a relatively low weight-average molecular weight, not only prevents adhesion at the surgical site but also effective in preventing adhesions over a wide area of the applied region, thereby accomplishing the present invention.

The scope of this invention is defined by the claims. Any references in the description to methods of treatment refer to the products of the present invention for use in a method for treatment.

Thus, the present invention is as follows.
[1-1] An anti-adhesion material comprising a biocompatible sponge-like laminate that is porous, includes first and second sponge-like layers containing low-endotoxin monovalent metal salts of alginic acid which are at least partially crosslinked with a curing agent, and is obtainable by commonly or individually lyophilizing the cured first and second layers of the laminate, wherein a weight-average molecular weight of the monovalent metal salt of alginic acid in the first layer is 30,000-300,000, a weight-average molecular weight of the monovalent metal salt of alginic acid in the second layer is 1,000-200,000, said low-endotoxin monovalent metal salts of alginic acid of the first and second layer have an endotoxin content of 500 Endotoxin Units (EU)/g or less measured using a limulus reagent, the weight- average molecular weights are measured by GPC-MALS method following a decrosslinking treatment, and the weight-average molecular weight of the monovalent metal salt of alginic acid in the first layer is higher than the weight- average molecular weight of the monovalent metal salt of alginic acid in the second layer; said anti-adhesion material having one or more of the following characteristics: (1) the Young's modulus determined based on a stress-strain curve acquired by subjecting a JIS K6251 Type 8-shaped tensile test piece to a tensile tester is 0.3- 300 MPa; (2) the strength at break determined based on the stress-strain curve acquired by subjecting the JIS K6251 Type 8-shaped tensile test piece to the tensile tester is 5-5000 kPa; and (3) the ratio of the strength at break to the Young's modulus of the sponge-like laminate determined based on a stress-strain curve acquired by subjecting a JIS K6251 Type 8-shaped tensile test piece to a tensile tester is 2-50.
[1-2] The anti-adhesion material according to [1-1] above, wherein either one of the first layer and the second layer contains the curing agent.
[1-3] The anti-adhesion material according to any one of [1-1] to [1-2] above, wherein both of the first layer and the second layer contain the curing agent.
[1-4] The anti-adhesion material according to any one of [1-1] to [1-3] above, wherein the total amount of the low-endotoxin monovalent metal salts of alginic acid used in the first layer and the second layer is in a range of 0.1 mg/cm²-3 mg/cm².
[1-5] The anti-adhesion material according to any one of [1-1] to [1-4] above, wherein
   the monovalent metal salts of alginic acid in the first layer and the second layer are sodium alginate or potassium alginate.
[1-6] The anti-adhesion material according to any one of [1-1] to [1-5] above, wherein
   the curing agent in the first layer and the second layer is at least one metal ionic compound selected from the group consisting of CaCl₂, CaSO₄, ZnCl₂, SrCl₂, FeCl₃, BaCl₂, CaHPO₄, calcium gluconate, calcium oxalate and calcium lactate.
[1-7] The anti-adhesion material according to any one of [1-1] to [1-6] above, which is for use in being applied such that the first layer faces the surface of a wound.
[1-8] The anti-adhesion material according to any one of [1-1] to [1-7] above, wherein
   the sponge-like laminate is sterilized by electron-beam and/or gamma-ray irradiation at an absorbed dose of 10 kGy-150 kGy, and/or by ethylene oxide gas.
[1-9] The anti-adhesion material according to either one of [1-8] above,
   wherein, in a dissolution test that uses elution of a monovalent metal salt of alginic acid in a phosphate buffer solution at pH 7.5 as an indicator, a proportion of the amount of the monovalent metal salt of alginic acid eluted from the first layer is less than 50% after an hour and less than 70% after 2 hours following the start of the measurement, when taking the amount of the monovalent metal salt of alginic acid eluted from the second layer as a base of 100%.
[1-10] The anti-adhesion material according to either one of [1-8] above,
   wherein, in a dissolution test that uses elution of a monovalent metal salt of alginic acid in a phosphate buffer solution at pH 7.5 as an indicator, the monovalent metal salt of alginic acid eluted from the first layer is 25 ± 10 wt% within an hour and 80 ± 10 wt% within 4 hours while the monovalent metal salt of alginic acid eluted from the second layer is 70 ± 10 wt% within an hour and 90 ± 10 wt% within 4 hours.
[1-11] The anti-adhesion material according to either one of [1-8] above,
   wherein, in a dissolution test that uses elution of a monovalent metal salt of alginic acid via an agarose gel in a phosphate buffer solution at pH 7.5 as an indicator, a proportion of the amount of the monovalent metal salt of alginic acid eluted from the first layer is 30-70% 2 days or later following the start of the measurement, when taking the amount of the monovalent metal salt of alginic acid eluted from the second layer as a base of 100%.
[1-12] The anti-adhesion material according to either one of [1-8] above,
   wherein, in a dissolution test that uses elution of a monovalent metal salt of alginic acid via an agarose gel in a phosphate buffer solution at pH 7.5 as an indicator, the monovalent metal salt of alginic acid eluted from the first layer is about 15 ± 5 wt% within 2 days and about 25 ± 10 wt% within 8 days while the monovalent metal salt of alginic acid eluted from the second layer is about 30 ± 8 wt% within 2 days and about 60 ± 10 wt% within 8 days.
[1-13] The anti-adhesion material according to any one of [1-1] to [1-12] above,
   wherein the sponge-like laminate is pressed.
[1-14] The anti-adhesion material according to any one of [1-1] to [1-13] above, which has one or more of the following characteristics:
   (1) when the sponge-like laminate is brought into contact with an agarose gel immersed in phosphate buffered saline for 2-6 hours, the weight increase of the sponge-like laminate is 200-50,000%, when taking the weight of the sponge-like laminate before it is brought into contact with the phosphate buffered saline as a base of 100%;
   (2) in a dissolution test that uses elution of a monovalent metal salt of alginic acid via an agarose gel in a phosphate buffer solution at pH 7.5 as an indicator, a proportion of the amount of the monovalent metal salt of alginic acid eluted from the first layer is 30-70% 2 days or later following the start of the measurement, when taking the amount of the monovalent metal salt of alginic acid eluted from the second layer as a base of 100%.
   (3) in a dissolution test that uses elution of a monovalent metal salt of alginic acid via an agarose gel in a phosphate buffer solution at pH 7.5 as an indicator, the monovalent metal salt of alginic acid eluted from the first layer is about 15 ± 5 wt% within 2 days and about 25 ± 10 wt% within 8 days while the monovalent metal salt of alginic acid eluted from the second layer is about 30 ± 8 wt% within 2 days and about 60 ± 10 wt% within 8 days.
[2-1] An anti-adhesion material according to any [1-14] for use in preventing an adhesion of the material to a wound of a subject by applying the first layer of the material to the wound so that it faces the surface of the wound.
[2-2] The anti-adhesion material for use according to [2-1] above, wherein either one of the first layer and the second layer contains the curing agent.
[2-3] The anti-adhesion material for use according to any one of [2-1] to [2-2] above, wherein both of the first layer and the second layer contain the curing agent.
[2-4] The anti-adhesion material for use according to any one of [2-1] to [2-3] above, wherein the total amount of the low-endotoxin monovalent metal salts of alginic acid used in the first layer and the second layer is in a range of 0.1 mg/cm²-3 mg/cm².
[2-5] The anti-adhesion material for use according to any one of [2-1] to [2-4] above, wherein the monovalent metal salts of alginic acid in the first layer and the second layer are sodium alginate or potassium alginate.
[2-6] The anti-adhesion material for use according to any one of [2-1] to [2-5] above, wherein the curing agent in the first layer and the second layer is at least one metal ionic compound selected from the group consisting of CaCl₂, CaSO₄, ZnCl₂, SrCl₂, FeCl₃, BaCl₂, CaHPO₄, calcium gluconate, calcium oxalate and calcium lactate.
[2-7] The anti-adhesion material for use according to any one of [2-1] to [2-6] above, wherein the sponge-like laminate is sterilized by electron-beam and/or gamma-ray irradiation at an absorbed dose of 10 kGy-150 kGy, and/or by ethylene oxide gas.
[3-1] A method for producing an anti-adhesion material according to [1-1] to [1-14] comprising the steps of: (1) curing a low-endotoxin monovalent metal salt of alginic acid having a weight- average molecular weight of 30,000-300,000 by using a curing agent; (2) on the monovalent metal salt of alginic acid obtained in (1), curing a low- endotoxin monovalent metal salt of alginic acid having a weight-average molecular weight of 1,000-200,000 by using a curing agent to obtain a laminate; and (3) lyophilizing the resulting laminate to obtain the sponge-like laminate, wherein the molecular weights are measured by GPC-MALS method, the sponge- like laminate includes a first sponge-like layer containing the low-endotoxin monovalent metal salt of alginic acid having a weight-average molecular weight of 30,000-300,000 and a second sponge-like layer containing the low-endotoxin monovalent metal salt of alginic acid having a weight-average molecular weight of 1,000-200,000, said low-endotoxin monovalent metal salts of alginic acid of the first and second layer have an endotoxin content of 500 Endotoxin Units (EU)/g or less measured using a limulus reagent, and the weight-average molecular weight of the monovalent metal salt of alginic acid in the first layer is higher than that in the second layer.
[3-2] The method for producing an anti-adhesion material according to any one of [3-above, wherein the sponge-like laminate is sterilized by electron-beam and/or gamma-ray irradiation at an absorbed dose of 10 kGy-150 kGy, and/or by ethylene oxide gas.
[3-3] The method for producing an anti-adhesion material according to any one of [3-1] to [3-2] above, wherein the curing agent is at least one metal ionic compound selected from the group consisting of CaCl₂, CaSO₄, ZnCl₂, SrCl₂, FeCl₃, BaCl₂, CaHPO₄, calcium gluconate, calcium oxalate and calcium lactate.
[4-1] A biocompatible sponge-like laminate that is porous and is obtainable by the following steps (1)-(3):
   (1) curing a low-endotoxin monovalent metal salt of alginic acid having a weight-average molecular weight of 30,000-300,000 by using a curing agent;
   (2) on the monovalent metal salt of alginic acid obtained in (1), curing a low-endotoxin monovalent metal salt of alginic acid having a weight-average molecular weight of 1,000-200,000 by using a curing agent to obtain a laminate; and
   (3) lyophilizing the resulting laminate to obtain the sponge-like laminate,
   wherein the molecular weights are measured by GPC-MALS method, the sponge-like laminate includes a first sponge-like layer containing the low-endotoxin monovalent metal salt of alginic acid having a weight-average molecular weight of 30,000-300,000 and a second sponge-like layer containing the low-endotoxin monovalent metal salt of alginic acid having a weight-average molecular weight of 1,000-200,000, said low-endotoxin monovalent metal salts of alginic acid of the first and second layer have an endotoxin content of 500 Endotoxin Units (EU)/g or less measured using a limulus reagent, and the weight-average molecular weight of the monovalent metal salt of alginic acid in the first layer is higher than that in the second layer, and wherein said sponge-like laminate having one or more of the following characteristics:
   (1) the Young's modulus determined based on a stress-strain curve acquired by subjecting a JIS K6251 Type 8-shaped tensile test piece to a tensile tester is 0.3-300 MPa;
   (2) the strength at break determined based on the stress-strain curve acquired by subjecting the JIS K6251 Type 8-shaped tensile test piece to the tensile tester is 5-5000 kPa; and
   (3) the ratio of the strength at break to the Young's modulus of the sponge-like laminate determined based on a stress-strain curve acquired by subjecting a JIS K6251 Type 8-shaped tensile test piece to a tensile tester is 2-50.
[4-2] The sponge-like laminate according to any one of [4-1] above, which is used as an anti-adhesion material.
[4-3] The sponge-like laminate according to any one of [4-1] to [4-2] above, wherein the sponge-like laminate is sterilized by electron-beam and/or gamma-ray irradiation at an absorbed dose of 10 kGy-150 kGy, and/or by ethylene oxide gas.
[4-4] The sponge-like laminate according to any one of [4-1] to [4-3] above, further comprising a step of pressing the laminate obtained in (3).
[4-5] The sponge-like laminate according to any one of [4-1] to [4-4] above, wherein the curing agent is at least one metal ionic compound selected from the group consisting of CaCl₂, CaSO₄, ZnCl₂, SrCl₂, FeCl₃, BaCl₂, CaHPO₄, calcium gluconate, calcium oxalate and calcium lactate.
[4-6] The sponge-like laminate according to any one of [4-1] to [4-5] above, wherein the sponge-like laminate has one or more of the following further characteristics:
   (4) when the sponge-like laminate is brought into contact with an agarose gel immersed in phosphate buffered saline for 2-6 hours, the weight increase of the sponge-like laminate is 200-50,000%, when taking the weight of the sponge-like laminate before it is brought into contact with the phosphate buffered saline as a base of 100%;
   (5) in a dissolution test that uses elution of a monovalent metal salt of alginic acid via an agarose gel in a phosphate buffer solution at pH 7.5 as an indicator, a proportion of the amount of the monovalent metal salt of alginic acid eluted from the first layer is 30-70% 2 days or later following the start of the measurement, when taking the amount of the monovalent metal salt of alginic acid eluted from the second layer as a base of 100%.
   (6) in a dissolution test that uses elution of a monovalent metal salt of alginic acid via an agarose gel in a phosphate buffer solution at pH 7.5 as an indicator, the monovalent metal salt of alginic acid eluted from the first layer is about 15 ± 5 wt% within 2 days and about 25 ± 10 wt% within 8 days while the monovalent metal salt of alginic acid eluted from the second layer is about 30 ± 8 wt% within 2 days and about 60 ± 10 wt% within 8 days.

### EFFECT OF THE INVENTION

The present invention can provide an anti-adhesion material that has at least one of the following advantages: highly effective in preventing adhesions; capable of suppressing both adhesion of a wound and *de novo* adhesion; has no adverse effect on a living body applied; does not interfere with healing of a wound; can be used for intestinal anastomosis or the like; allows easy application via a trocar upon an endoscopic surgery; capable of being reapplied to adjust the applied position; excellent in production efficiency; suitable for mass production; and the like.

### BRIEF DESCRIPTION OF THE DRAWINGS

[Figure 1] A view showing an example of an anti-adhesion material.
[Figure 2] A diagram showing evaluation of the dissolution rates of the respective layers of the anti-adhesion material by an immersing approach.
[Figure 3] A diagram showing evaluation of the dissolution rates of the respective layers of the anti-adhesion material by an applying approach.
[Figure 4] Diagrams showing evaluations of adhesion formation in partially resected hepatic models. (A) Number of individuals that formed adhesions on the resected surfaces, (B) grade of the resected surfaces, and (C) extent of the resected surfaces (mm). **p<0.01, *p<0.05.
[Figure 5] Diagrams showing evaluations of adhesion formation in the partially resected hepatic models. (A) Number of individuals that formed adhesions on the unresected surfaces, (B) grade of the unresected surfaces, and (C) extent of the unresected surfaces (mm). **p<0.01, *p<0.05.
[Figure 6] Diagrams showing evaluations of changes in body weight and spleen weight of the partially resected hepatic models. (A) Change in body weight, and (B) spleen weight.
[Figure 7] Diagrams showing evaluations of adhesion formation in Pean clamp-resected hepatic models. (A) Number of individuals that formed adhesions on the resected surfaces, (B) grade of the resected surfaces, and (C) extent of the resected surfaces (mm). **p<0.01, *p<0.05.
[Figure 8] Diagrams showing evaluations of adhesion formation in the Pean clamp-resected hepatic models. (A) Number of individuals that formed adhesions on the unresected surfaces, (B) grade of the unresected surfaces, and (C) extent of the unresected surfaces (mm). **p<0.01, *p<0.05.
[Figure 9] Diagrams showing evaluation of changes in body weight and spleen weight of the Pean clamp-resected hepatic models. (A) Change in body weight, and (B) spleen weight.
[Figure 10] A diagram showing results from sponge swelling tests before and after pressing.
[Figure 11] Diagrams showing changes in (A) the height of the tip of each test piece and (B) the angle thereof with respect to the test board with time after spraying.
[Figure 12] A diagram showing results from a water sorption test of layered sponges after sterilization.

### MODE FOR CARRYING OUT THE INVENTION

Hereinafter, the present invention will be described in detail. The following embodiments are illustrations of the present invention, and the present invention can be carried out in various embodiments without departing from the gist thereof.

### 1. Adhesion prevention

"Adhesions" refer to a state where surfaces of tissues that should be separated from each other are connected or fused to each other via fibrous tissue. Adhesions are caused by an injury generated on a surface of a tissue upon a surgical operation, inflammation caused by an injury, and inflammation caused by drying of a tissue surface upon a surgical operation. Adhesions are formed in association with such injury or inflammation upon which an exudate containing fibrin is emitted on a surface of a tissue, where this exudate is organized such that the tissue surfaces are connected or fused.

"Adhesion prevention" means to reduce formation of adhesions. Adhesion prevention does not necessarily require complete prevention of adhesion formation, and may apply as long as formation of adhesion is prevented as compared to a state where an anti-adhesion material of the present invention is not applied. Specifically, "adhesion prevention" may also refer to as amelioration of adhesions, which may mean, for example, amelioration of at least one selected from frequency, area and degree of the adhesions. "Adhesion prevention" may be, for example, lowering of the average adhesion grade as compared to the average adhesion grade without application of the anti-adhesion material of the present invention when adhesion grade is evaluated as described in the example. Alternatively, "adhesion prevention" may be, for example, lowering of the average adhesion extent as compared to the average adhesion extent without application of the anti-adhesion material of the present invention when adhesion extent is evaluated as described in the example. "Adhesion prevention" preferably refers to prevention of adhesion resulting from a surgical operation, and more preferably refers to prevention of peritoneal adhesion resulting from a surgical operation. Specifically, "adhesion prevention" preferably refers to prevention of an adhesion following a surgery.

In addition, as described in the examples, adhesions targeted include an adhesion of a resected site of an organ targeted by a surgery and a *de novo* adhesion (adhesions formed at variety of sites other than the surgical site, in the periphery, the body cavity such as the abdominal cavity and the body).

### 2. Anti-adhesion material

The present invention provides an anti-adhesion material (hereinafter, sometimes referred to as an "anti-adhesion material A") comprising a biocompatible sponge-like laminate that includes first and second sponge-like layers containing low-endotoxin monovalent metal salts of alginic acid which are at least partially crosslinked with a curing agent, wherein the first sponge-like layer contains a low-endotoxin monovalent metal salt of alginic acid with a relatively high weight-average molecular weight and the second sponge-like layer contains a low-endotoxin monovalent metal salt of alginic acid with a relatively low weight-average molecular weight. The weight-average molecular weights are measured by GPC-MALS method following a decrosslinking treatment, for example, following dissolution in a solution containing a chelating agent.

The monovalent metal salt of alginic acid used in the respective layers may be a combination of a plurality of monovalent metal salts of alginic acid having different average molecular weights. The number of the monovalent metal salts of alginic acid to be combined is not particularly limited, and may be, for example, 2, 3, 4, 5, 6, 7, 8, 9, 10 or more in one layer. The monovalent metal salts of alginic acid may be a combination of different types of salts having different weight-average molecular weights or a combination of the same type of salts having different weight-average molecular weights. The ratio of the combination is also not particularly limited. For example, when two types of salts are combined, the ratio thereof may be 1:100-100:1, 1:50-50:1, 1:25-25:1, 1:10-10:1, 1:5-5:1, 1:4-4:1, 1:3-3:1, 1:2-2:1, 1:1 or the like.

Herein, the sign "-" used for a numerical range represents "the lower limit value to the upper limit value" where the numerical values on both sides of the sign are inclusive in said range.

The anti-adhesion material A contains low-endotoxin monovalent metal salts of alginic acid having different molecular weights in the first layer and the second layer of the sponge-like laminate. The weight-average molecular weight of the monovalent metal salt of alginic acid in the first layer is higher than that in the second layer. A dissolution rate of a layer containing a monovalent metal salt of alginic acid becomes slower with a larger weight-average molecular weight of the monovalent metal salt of alginic acid whereas the dissolution rate becomes faster with a smaller weight-average molecular weight. Accordingly, for example, for an application to an intraperitoneal wound, the anti-adhesion material A of the present invention is applied such that the first layer faces the wound while the second layer faces the abdominal cavity, expecting that the first layer remains at the wound while the second layer dissolves relatively faster to suppress adhesions generally in the abdominal cavity.

Furthermore, the present invention provides an anti-adhesion material (an "anti-adhesion material B") comprising a biocompatible sponge-like laminate which includes a first layer and a second layer each containing a low-endotoxin monovalent metal salt of alginic acid, which are at least partially crosslinked with a curing agent, wherein dissolution rates of the first layer and the second layer are different. Specifically, the dissolution rate of the first layer is slower than that of the second layer.

In a case of the anti-adhesion material B, the proportion of the amount of the monovalent metal salt of alginic acid eluted from the first layer is preferably less than 50% after an hour and less than 70% after 2 hours following the start of the measurement in a dissolution test that uses elution of the monovalent metal salt of alginic acid as an indicator, when taking the amount of the monovalent metal salt of alginic acid eluted from the second layer as a base of 100%. The details of the dissolution test will be described in Example 2 below.

In another preferable example of the anti-adhesion material B, the monovalent metal salt of alginic acid eluted from the first layer is 25 ± 10 wt% within an hour and 80 ± 10 wt% within 4 hours while the monovalent metal salt of alginic acid eluted from the second layer is 70 ± 10 wt% within an hour and 90 ± 10 wt% within 4 hours, in a dissolution test that uses elution of the monovalent metal salt of alginic acid as an indicator. The details of the dissolution test will be described in Example 2 below.

In another preferable example of the anti-adhesion material B, the proportion of the amount of the monovalent metal salt of alginic acid eluted from the first layer is about 30% to about 70%, preferably about 40% to about 60% and more preferably about 44% to about 55% 2 days or later following the start of the measurement in a dissolution test that uses elution of the monovalent metal salt of alginic acid via an agarose gel as an indicator, when taking the amount of the monovalent metal salt of alginic acid eluted from the second layer as a base of 100%. The details of the dissolution test will be described in Example 2-2 below.

In another preferable example of the anti-adhesion material B, the monovalent metal salt of alginic acid eluted from the first layer is about 15 ± 5 wt%, in particular about 16 ± 2 wt% within 2 days and about 25 ± 10 wt%, in particular about 33 ± 7 wt% within 8 days while the monovalent metal salt of alginic acid eluted from the second layer is about 30 ± 8 wt%, in particular about 31 ± 6 wt% within 2 days and about 60 ± 10 wt%, in particular about 60 ± 7 wt% within 8 days, in a dissolution test that uses elution of the monovalent metal salt of alginic acid via an agarose gel as an indicator. The details of the dissolution test will be described in Example 2-2 below.

Herein, the "anti-adhesion material A" and the "anti-adhesion material B" may sometimes collectively be referred to as an "anti-adhesion material". The "first layer" refers to a layer that becomes a lower layer when the sponge-like laminate is applied to a subject, namely, a layer that makes contact with a surface of a tissue to be applied in the subject. The "second layer" refers to a layer that becomes an upper layer when the sponge-like laminate is applied to a subject, namely, a layer that does not make contact with the surface of the tissue to be applied in the subject. The phrase "biocompatible" means that it can be placed on a surface of a tissue to be applied as a medical material.

A clear boundary may or may not be formed between the first layer and the second layer of the biocompatible sponge-like laminate that is used as an anti-adhesion material. For example, a clear boundary may not be formed in the laminate because the component contained in the first layer and the component contained in the second layer may be mixed together in the vicinity of the boundary between the first layer and the second layer during the manufacturing process. Furthermore, this laminate may have, in addition to the above-described first and second layers, a third layer containing any component, or may have a multilayer structure. In addition, the sponge-like laminate may also comprise a structure in which there is no clear boundary between the layers and the molecular weight gradually increases or decreases in a continuous way.

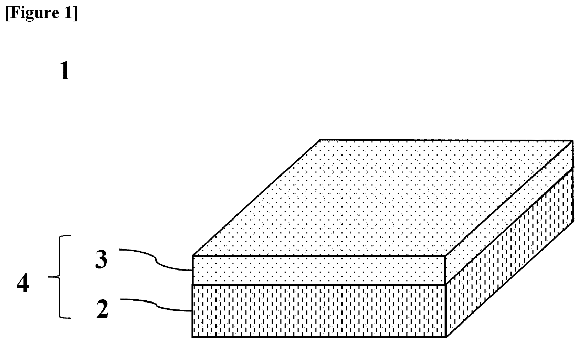

An exemplary anti-adhesion material is shown in Figure 1. An anti-adhesion material 1 comprises a sponge-like laminate 4 including a first layer 2 and a second layer 3. Each of the first layer 2 and the second layer 3 is a sponge-like layer. The term "sponge-like" refers to a porous state.

The shape of the biocompatible sponge-like laminate is not particularly limited and may suitably be selected considering the area, shape, unevenness and the like of the surface to be applied. The shape of the sponge-like laminate may be, for example, a plate as shown in Figure 1, or it may have a shape such as a disc, a cylinder, a rectangular cuboid or the like. Preferably, it is a plate or a disc. If it is a plate or a disc, the size of the plate or the disc is not particularly limited since the anti-adhesion material can further be cut in accordance with the area, shape, unevenness or the like of the surface applied before being applied to the surface. For example, where a shape of a plate is expressed by length x width x height (thickness), the length and the width are not particularly limited while the height (thickness) is preferably 0.2 mm-30 mm, more preferably 0.3 mm-15 mm, and still more preferably 0.5 mm-10 mm. Yet still more preferably, in addition to such a height (thickness), the length and the width are 1 mm-300 mm x 1 mm-300 mm, particularly preferably 3 mm-200 mm x 3 mm-200 mm, and more preferably 5 mm-150 mm x 5 mm-150 mm. The thickness may not be uniform and the laminate may have a slope structure with one thicker end and the other thinner end.

A sponge-like laminate of the anti-adhesion material of the present invention is highly flexible and hard to break as compared to Seprafilm (trade name).

In some aspects, the sponge-like laminate is pressed. "Pressing" will be described hereinbelow. A pressed sponge-like laminate has a height (thickness) of preferably 0.01 mm-5 mm, more preferably 0.02 mm-3 mm, and still more preferably 0.03 mm-1.5 mm. More preferably, in addition to such a height (thickness), the length and the width are 1 mm-300 mm x 1 mm-300 mm, particularly preferably 3 mm-200 mm x 3 mm-200 mm, and more preferably 5 mm-150 mm x 5 mm-150 mm. In some aspects, the thickness after pressing is uniform.

The sponge-like laminate of the anti-adhesion material of the present invention may have the following physical characteristics.

### (1) Young's modulus

The Young's modulus of the sponge-like laminate determined based on a stress-strain curve acquired by subjecting a JIS K6251 Type 8-shaped tensile test piece to a tensile tester may be, for example, 0.3-300 MPa, preferably 0.4-250 MPa, more preferably 0.5-200 MPa, and particularly preferably 1-180 MPa. The Young's modulus can be increased by pressing the laminate.

### (2) Strength at break

The strength at break of the sponge-like laminate determined based on a stress-strain curve acquired by subjecting a JIS K6251 Type 8-shaped tensile test piece to a tensile tester may be, for example, 5-5000 kPa, preferably 10-4800 kPa, more preferably 15-4500 kPa, and particularly preferably 20-4000 kPa. The strength at break can be increased by pressing the laminate.

### (3) Strength at break/Young's modulus ratio

The ratio of the strength at break to the Young's modulus of the sponge-like laminate determined based on a stress-strain curve acquired by subjecting a JIS K6251 Type 8-shaped tensile test piece to a tensile tester (Strength at break (kPa)/Young's modulus (MPa)) is, for example, 2-50, preferably 3-45, more preferably 5-40, and particularly preferably 10-35.

### (4) Amount of water absorption

When the sponge-like laminate (lyophilized) is brought into contact with an agarose gel immersed in phosphate buffered saline for 2-6 hours, the weight increase of the sponge-like laminate is, for example, 200-50,000%, preferably 500-30,000%, more preferably 1,000-20,000% and particularly preferably 1,500-15000% when taking the weight of the sponge-like laminate before it is brought into contact with the phosphate buffered saline as a base of 100%.

### 3. Monovalent metal salt of alginic acid

A "monovalent metal salt of alginic acid" is a water-soluble salt that is formed through ion exchange between a hydrogen atom of carboxylic acid at position 6 of alginic acid and a monovalent metal ion such as Na⁺ or K⁺. Specific examples of monovalent metal salts of alginic acid include sodium alginate and potassium alginate, while sodium alginate that can be obtained as a commercially available product is particularly preferable. A solution of a monovalent metal salt of alginic acid forms a gel when mixed with a curing agent.

"Alginic acid" used in the present invention is a biodegradable polymeric polysaccharide, which is a polymer resulting from linear polymerization of two types of uronic acids called D-mannuronic acid (M) and L-guluronic acid (G). More specifically, alginic acid is a block copolymer which has a homopolymer fraction of D-mannuronic acid (MM fraction), a homopolymer fraction of L-guluronic acid (GG fraction) and a fraction of randomly arranged D-mannuronic acids and L-guluronic acids (MG fraction), arbitrarily linked together. A composite ratio of D-mannuronic acid to L-guluronic acid (M/G ratio) of alginic acid varies primarily according to the type of the biological origin such as seaweed, and is affected by the habitat of said biological origin and seasons. The M/G ratio widely ranges from about 0.4 that is rich in G to about 5 that is rich in M.

Since a monovalent metal salt of alginic acid is a polymeric polysaccharide, it is difficult to accurately determine the molecular weight thereof. Thus, a molecular weight of a polymeric substance derived from a natural origin is known to vary depending on the measurement method.

An absolute weight-average molecular weight can be measured by GPC-MALS method.

In a specific aspect, a weight-average molecular weight of a monovalent metal salt of alginic acid that can be used as a feedstock of the first layer of the sponge-like laminate as measured by GPC-MALS method is, for example, 30,000-300,000, preferably 80,000-280,000, more preferably 100,000-270,000, still more preferably 150,000-260,000, and particularly preferably 170,000-250,000. In addition to said first layer, a weight-average molecular weight of the second layer as measured by GPC-MALS method is, for example, 1,000-200,000, preferably 1,500-180,000, more preferably 2,000-150,000, still more preferably 2,500-120,000, and particularly preferably 3,000-100,000.

A monovalent metal salt of alginic acid having a weight-average molecular weight in the above-described range as measured by GPC-MALS method can be used as a feedstock of the first layer of a sponge-like laminate so as to optimize the gelation rate of the monovalent metal salt of alginic acid upon manufacturing the first layer of the sponge-like laminate, thereby optimizing the quality and the manufacturing time of the first layer.

In some specific aspects, after sterilization by, for example, electron-beam and/or gamma-ray irradiation, a weight-average molecular weight of the first layer of the sponge-like laminate as measured by GPC-MALS method is, for example, 30,000-80,000, preferably 40,000-75,000, more preferably 42,000-73,000, still more preferably 43,000-72,000, and particularly preferably 44,000-71,000. In addition to said first layer, a weight-average molecular weight of the second layer as measured by GPC-MALS method is, for example, 1,000-70,000, preferably 1,000-65,000, more preferably 2,000-60,000, still more preferably 2,500-55,000, and particularly preferably 3,000-50,000.

The weight-average molecular weight of a monovalent metal salt of alginic acid which is at least partially crosslinked with a curing agent can be determined by GPC-MALS method following any decrosslinking treatment as a weight average molecular weight of the monovalent metal salt of alginic acid that is not crosslinked. For example, the decrosslinking treatment may be conducted by dissolving in any chelating agent, for example, a solution of a chelating agent such as EDTA (ethylenediaminetetraacetic acid) or phytic acid. Preferably, EDTA is used as the chelating agent.

The weight-average molecular weight of the monovalent metal salt of alginic acid in the first layer of the sponge-like laminate is higher than that in the second layer. The weight-average molecular weight of the monovalent metal salt of alginic acid in the first layer of the feedstock of the sponge-like laminate or of the sponge-like laminate is higher than that in the second layer, for example, by 1,000-1,000,000, preferably by 2,000-500,000, and more preferably by 3,000-300,000.

In general, a measurement error of 10-20 wt% may be expected when a molecular weight of a polymeric polysaccharide is calculated by a technique described above. Thus, the value of 10,000 may vary in a range of about 8,000-12,000, the value of 100,000 may vary in a range of about 80,000-120,000, the value of 200,000 may vary in a range of about 160,000-240,000, the value of 400,000 may vary in a range of about 320,000-480,000, and the value of 500,000 may vary in a range of about 400,000-600,000.

A molecular weight of alginic acid may be measured according to a common method. Typical conditions for employing GPC-MALS for molecular weight measurement are described herein in Example 1. As the detector, for example, a RI detector and a light scattering detector (MALS) may be used.

Although alginic acid extracted from a brown alga initially has a large molecular weight, the molecular weight thereof gradually becomes smaller during the processes of heat drying, purification and the like. Alginic acids having different molecular weights can be produced by techniques like management of conditions such as the temperature or the like during the production steps, selection of the brown alga as the raw material, fractionation based on molecular weights during the production steps, and the like. Furthermore, alginic acid having a molecular weight of interest can also be obtained by mixing with alginic acid from other lot having a different molecular weight.

A monovalent metal salt of alginic acid used in the present invention is subjected to a low endotoxin treatment. The low endotoxin treatment can be performed according to a known method or a method pursuant thereto. For example, the treatment can be carried out according to the method of Suga et al. involving purification of sodium hyaluronate (see, for example, Japanese Unexamined Patent Application Publication No. Heisei 9-324001), the method of Yoshida et al., involving purification of β1,3-glucan (see, for example, Japanese Unexamined Patent Application Publication No. Heisei 8-269102), the method of William et al. involving purification of a biopolymer salt such as alginate or gellan gum (see, for example, Japanese Unexamined Patent Application Publication (Translation of PCT Publication) No. 2002-530440), the method of James et al. involving purification of a polysaccharide (see, for example, pamphlet of International Publication No. 93/13136), the method of Lewis et al. (see, for example, specification of US Patent No. 5589591), the method of Hermanfranck et al. involving purification of alginate (see, for example, Appl Microbiol Biotechnol (1994) 40:638-643), or a method pursuant thereto. The low endotoxin treatment of the present invention is not limited thereto, and can be carried out by a known method such as washing, filtration with a filter (e.g., an endotoxin-removing filter or an electrically-charged filter), ultrafiltration, purification with a column (e.g., an endotoxin adsorption affinity column, a gel filtration column or an ion-exchange resin column), adsorption to a hydrophobic substance, a resin or activated charcoal, a treatment with an organic solvent (extraction with an organic solvent, deposition/precipitation through addition of an organic solvent, or the like), a surfactant treatment (see, for example, Japanese Unexamined Patent Application Publication No. 2005-036036), or an appropriate combination thereof. The steps of these treatments may appropriately be combined with a known method such as centrifugation. Preferably, the treatment is suitably selected according to the type of the alginic acid.

An endotoxin level can be confirmed according to a known method. For example, it can be measured by a method using a limulus reagent (LAL), or a method using Endospecy (registered trademark) ES-24S set (Seikagaku Corporation).

Although a method for treating endotoxin of a monovalent metal salt of alginic acid used in the present invention is not particularly limited, the resulting endotoxin content of a bioabsorbable polysaccharide is 500 endotoxin unit (EU)/g or less, preferably 100 EU/g or less, more preferably 50 EU/g or less, and still more preferably 30 EU/g or less upon an endotoxin measurement using a limulus reagent (LAL). Sodium alginate that has been subjected to a low endotoxin treatment is available, for example, as a commercially available product such as Sea Matrix (registered trademark) (Mochida Pharmaceutical Co., Ltd.) and PRONOVA^{™} UP LVG (FMC BioPolymer).

The amount of the monovalent metal salt of alginic acid used in the sponge-like laminate may appropriately be selected considering the effect of preventing an adhesion. The total amount of the monovalent metal salts of alginic acid used in the first layer and the second layer of the sponge-like laminate may be, for example, 0.1 mg/cm²-10.0 mg/cm², preferably 0.1 mg/cm²-3.0 mg/cm², more preferably 0.5 mg/cm²-2.5 mg/cm², still more preferably 1.8 mg/cm²-2.2 mg/cm², and particularly preferably 2.0 mg/cm². If the total amount of the monovalent metal salts of alginic acid used in the first layer and the second layer of the sponge-like laminate is 1.0 mg/cm²-3.0 mg/cm², a higher adhesion prevention effect can be expected. The risk of adverse events such as accumulation in the living body or enlargement of a specific organ is small if the amount used is 10.0 mg/cm² or less while a satisfactory adhesion prevention effect can be expected if the amount used is 0.1 mg/cm² or more.

The ratio of the amounts of the monovalent metal salts of alginic acid used in the first layer and the second layer (weight ratio) is preferably 1:20-20:1, more preferably 1:5-5:1, still more preferably 1:3-3:1, and particularly preferably 1:2-2:1.

### 4. Curing agent (crosslinking agent)

The anti-adhesion material may contain a curing agent in either one of the first layer and the second layer (in other words, either one of the first layer and the second layer may not contain a curing agent), or both of the first layer and the second layer may contain a curing agent.

Alternatively, both of the first and second layers of the anti-adhesion material may not contain a curing agent.

In some aspects, the first layer and the second layer are at least partially crosslinked with a curing agent.

The curing agent causes hardening by crosslinking a solution of the monovalent metal salt of alginic acid. Examples of the curing agent include bivalent or higher metal ion compounds of Ca²⁺, Mg²⁺, Ba²⁺, Sr²⁺, Zn²⁺ and Fe³⁺ and crosslinking reagents that have two to four amino groups within their molecules. More specifically, examples of bivalent or higher metal ion compounds include inorganic metal salts such as CaCl₂, MgCl₂, CaSO₄, ZnCl₂, FeCl₃, BaCl₂, SrCl₂ and dibasic calcium phosphate (CaHPO₄), organic acid metal salts such as calcium gluconate, calcium oxalate, calcium lactate and the like (preferably, CaCl₂, CaSO₄, ZnCl₂, SrCl₂, FeCl₃, BaCl₂, dibasic calcium phosphate (CaHPO₄), calcium gluconate, etc.), while examples of crosslinking reagents having two to four amino groups within their molecules include diaminoalkanes optionally having a lysyl group (-COCH(NH₂)-(CH₂)₄-NH₂) on a nitrogen atom, that is, diaminoalkane and derivatives thereof that form lysyl amino groups by substituting an amino group with a lysyl group, specific examples being diaminoethane, diaminopropane and N-(lysyl)-diaminoethane.

Preferably, the amount of the curing agent used in the first layer and the second layer is suitably adjusted in accordance with the amount and the molecular weight of the monovalent metal salt of alginic acid used, the desired curing time, and the like. In a case where a curing agent is used, the amount of the curing agent used in the first layer is, for example, 0.1 µmol/cm²-100 µmol/cm², and preferably 0.5 µmol/cm²-2.0 µmol/cm². In a case where a curing agent is used, the amount of the curing agent used in the second layer is, for example, 0.1 µmol/cm²-10 µmol/cm², and preferably 0.6 µmol/cm²-2.4 µmol/cm². Furthermore, the curing time of each layer can be extended by reducing the amount of the curing agent used (concentration used), whereas the curing time of each layer can be shortened by increasing the amount of the curing agent used (concentration used).

### 5. Method for producing anti-adhesion material

An anti-adhesion material comprising a biocompatible sponge-like laminate or a biocompatible sponge-like laminate may be produced, for example, through the following steps:
(1) curing a low-endotoxin monovalent metal salt of alginic acid having a weight-average molecular weight of 10,000-2,000,000 by using a curing agent;
(2) on the monovalent metal salt of alginic acid obtained in (1),, curing a low-endotoxin monovalent metal salt of alginic acid having a weight-average molecular weight of 1,000-1,000,000 by using a curing agent to obtain a laminate; and
(3) lyophilizing the resulting laminate to obtain the sponge-like laminate,

In step (1) above, first, a solution of a low-endotoxin monovalent metal salt of alginic acid having a weight-average molecular weight of 10,000-2,000,000 (hereinafter, referred to as a "first alginic acid salt") and a solution of a curing agent are prepared. The solution of the first alginic acid salt and the solution of the curing agent can be prepared according to a known method or a method pursuant thereto. While the solvent can be any solvent as long as it is biocompatible, it is preferably an aqueous solvent, for example, purified water, pure water (e.g., distilled water, ion-exchanged water), Milli-Q water, physiological saline, phosphate buffered saline or DMSO, and more preferably pure water. The solvent is preferably sterilized and has been subjected to a low endotoxin treatment.

Then, the solution of the first alginic acid salt and the solution of the curing agent can be mixed to cure the first alginic acid salt.

In step (2) above, first, a solution of a low-endotoxin monovalent metal salt of alginic acid having a weight-average molecular weight of 1,000-1,000,000 (hereinafter, referred to as a "second alginic acid salt") and a solution of a curing agent are prepared. The solution of the second alginic acid salt and the solution of the curing agent can be prepared according to a known method or a method pursuant thereto. The solvent is the same as that described for step (1) above.

Then, the solution of the second alginic acid salt and the solution of the curing agent can be mixed to cure the second alginic acid salt.

In step (3) above, the laminate obtained in step (2) is lyophilized to obtain the sponge-like laminate. Lyophilizing can be carried out by a known method. Conditions for lyophilizing can suitably be adjusted, and lyophilizing may include a primary drying step, a secondary drying step and the like.

Through these steps, a biocompatible sponge-like laminate including a first sponge-like layer containing a first alginic acid salt and a curing agent and a second sponge-like layer containing a second alginic acid salt and a curing agent, wherein the weight-average molecular weight of the monovalent metal salt of alginic acid in the first layer is higher than that in the second layer, as well as an anti-adhesion material comprising said sponge-like laminate can be obtained.

According to the above-described method, the first sponge-like layer containing the first alginic acid salt and the curing agent is prepared at first, and then the second sponge-like layer containing the second alginic acid salt and the curing agent is prepared thereon.

An anti-adhesion material comprising a sponge-like laminate having a desired size, height and shape can be obtained by using a vessel, a mold, a substrate, a porous membrane, a non-woven fabric, a woven fabric or the like having the desired size, height and shape upon curing the first alginic acid salt and the second alginic acid salt.

The curing time of the first alginic acid salt and the second alginic acid salt can be extended by a technique such as decreasing the molecular weight of the alginic acid salt used, decreasing the concentration of the curing agent or decreasing the concentration of the alginic acid salt, whereas the curing time can be shortened by a technique such as increasing the molecular weight of the alginic acid salt used, increasing the concentration of the curing agent or increasing the concentration of the alginic acid salt. Although longer curing time enhances operability such as filling the curing alginic acid salt that is still undergoing curing into a vessel or a mold, the production efficiency may be affected if the curing time is too long.

Furthermore, the sponge-like laminate of the anti-adhesion material is preferably subjected to a sterilization treatment. Examples of sterilization include, but not limited to, gamma-ray sterilization, electron-beam sterilization, ethylene oxide gas sterilization, ethanol sterilization, low-temperature hydrogen peroxide gas plasma sterilization, vaporized hydrogen peroxide sterilization, and formaldehyde gas sterilization. More preferably, the anti-adhesion material is subjected to a sterilization treatment by electron-beam and/or gamma-ray irradiation. A polymeric material is subjected to an irradiation treatment with a gamma-ray, an electron beam or the like so as to preferably obtain a highly biocompatible medical material that allows controlled retention in the body (see, for example, Japanese Unexamined Patent Application Publication No. 2000-237294).

Examples of the irradiation conditions upon the electron-beam and/or gamma-ray sterilization include an absorbed dose of 10 kGy-150 kGy, more preferably 20 kGy-100 kGy, and still more preferably 40 kGy-80 kGy. In another preferable aspect, examples of the irradiation conditions upon the electron-beam and/or gamma-ray sterilization include an absorbed dose of 20 kGy-80 kGy, 20 kGy-60 kGy or 40 kGy-60 kGy. Electron-beam sterilization is more favorable than gamma-ray sterilization.

In another preferable aspect, the sponge-like laminate of the anti-adhesion material may be subjected to a sterilization treatment using ethylene oxide gas (EOG) according to a common method.

In yet another aspect, the sponge-like laminate of the anti-adhesion material may be produced from a sterilized or aseptic material under aseptic conditions. A sponge-like laminate produced as such may also be provided in an aseptic state without a sterilization treatment.

Some aspects further comprise a step of pressing the laminate obtained in step (4) or the like above. Pressing can be carried out by holding and applying pressure on the laminate manually or with a press machine. Generally employed steps such as compression and thinning are also included as pressing of the present invention. Examples of the pressure adopted for pressing include 1 kPa-100 MPa, more preferably 10 kPa-80 MPa, and still more preferably 100 kPa-60 Mpa. Manual pressing is carried out with a means that can apply uniform pressure onto the laminate by pressing it with a hand, for example, an acrylic ruler, an acrylic plate, a glass plate, a metal plate or the like. Moreover, an example of the press machine used includes a hot press machine (AH-1T from AS ONE Corporation).

The physical property values of the obtained sponge-like laminate including tensile strength, tear strength, degradability (solubility), water absorption rate (amount of water absorbed), deformation (viscoelasticity), air permeability, smoothness, and the like can be measured by a common method (for example, a technique defined by JIS, etc.).

### 6. Usage

The anti-adhesion material is used in being applied to a subject in need of adhesion prevention. Preferably, the anti-adhesion material remains on the applied site usually for about a week that is necessary for exhibiting the adhesion prevention effect, then absorbed and decomposed and eventually metabolized/excreted in about 1-2 months, and thus it is highly safety.

The anti-adhesion material may also be applied to a surface of a wound, for example, a surface of a tissue involved in a surgical operation.

A "tissue involved in a surgical operation" refers to a tissue that has a wound on its surface due to the surgical operation, or a tissue that has inflammation or that has a risk of inflammation due to drying of the surface due to a surgical operation. A tissue involved in a surgical operation is preferably an organ wrapped in peritoneum (for example, stomach, jejunum, ileum, appendix, colon, liver, gallbladder, spleen, duodenum, uterus, fallopian tube, ovary, abdominal wall and pancreas), an organ covered with a pleura (lung, chest wall), an organ covered with pericardium (heart, pericardium sac) or the like. An anti-adhesion material of a preferable aspect of the present invention is capable of effectively preventing a serious adhesion such as an adhesion occurring after hepatic resection.

Furthermore, to "apply" means to place an anti-adhesion material on a surface of a wound (for example, a surface of a tissue involved in a surgical operation). Specifically, for example, the anti-adhesion material is placed on a surface of a wound (for example, a surface of a tissue involved in a surgical operation) such that the surface of the first layer of the sponge-like laminate makes contact with the surface of the wound (for example, the surface of the tissue) while the surface of the second layer faces the opposite side (for example, the serous membrane side) of the surface of the wound (for example, the surface of the tissue). Examples of such a tissue include, but not limited to, tissues covered with a serous membrane (for example, stomach, jejunum, ileum, appendix, colon, liver, gallbladder, spleen, duodenum, pancreas, uterus, fallopian tube, ovary, greater omentum, mesentery and abdominal wall in the abdominal cavity, and lung, heart, pericardium sac and chest wall in the thoracic cavity, etc.). Accordingly, the anti-adhesion material can be placed such that the surface of the first layer of the sponge-like laminate makes contact with the surface of a wounded tissue which is directly targeted by a surgical operation or the like, or may be placed such that the surface of the first layer makes contact with the surface of a wounded body wall that has been invaded to provide access to said tissue. For example, in a surgical operation that involves a large invasion area at the site of skin incision as in an abdominal operation, upon closing the incision, the anti-adhesion material can be used such that the surface of the first layer of the sponge-like laminate makes contact with the skin incision (the body wall side).

According to the size of the incision and the degree of the invasion, a plurality of sponge-like laminates can be used upon a single operation. If a plurality of sponge-like laminates are to be used, all of the sponge-like laminates may be placed such that the surfaces of their first layers make contact with the surface of the wound of the target tissue (the organ side), or all of the sponge-like laminates may be placed such that the surfaces of their first layers make contact with the skin incision (the body wall side), some of the sponge-like laminates may be placed such that the surfaces of their first layers make contact with the surface of the target tissue on the wound side (the organ side), while other sponge-like laminates may be placed such that the surfaces of their first layer make contact with the skin incision (the body wall side). Since the first layer of the sponge-like laminate has a relatively high weight-average molecular weight, it remains on the surface of the wounded tissue without being decomposed for a sufficient amount of time to prevent adhesion, thereby serving as a physical barrier for the wounded surface. Meanwhile, since the second layer of the sponge-like laminate has a relatively low weight-average molecular weight, it melts and spreads rapidly to exert adhesion prevention for the surface without a wound.

Preferably, the sponge-like laminate of the anti-adhesion material is highly flexible and hard to break as compared to Seprafilm (trade name). Therefore, in a preferable aspect, application of the anti-adhesion material is not limited to the surface of the tissue to be applied and, for example, it can also be used to wrap around an intestinal tract upon intestinal anastomosis. In another preferable aspect, it can easily be inserted through a pathway for putting a surgical instrument in and out upon a surgical operation using an endoscope in a subject. In yet another preferable aspect, the anti-adhesion material can be reapplied.

Preferably, the sponge laminate of the anti-adhesion material can be applied to a wider range of targets for adhesion prevention compared to INTERCEED (trade name).

Preferably, the anti-adhesion material is prepared in a size appropriate for the area, shape, unevenness and the like of a surface to be applied, and applied to the surface of the tissue involved in a surgical operation for adhesion prevention. A "subject" may be human or an organism other than human, for example, a bird or a non-human mammal (for example, bovine, monkey, cat, mouse, rat, guinea pig, hamster, ferret, pig, dog, rabbit, sheep, goat or horse).

Since the sponge laminate of the anti-adhesion material can be made compact especially if the sponge laminate is pressed, the anti-adhesion material can be applied to the affected area relatively easily, for example, via a trocar or the like upon an endoscopic surgery. Thereafter, the anti-adhesion material applied to the affected area preferably absorbs moisture present in the affected area or moisture applied to the affected area to restore the thickness.

Preferably, the anti-adhesion material can be used safely in a subject just like Seprafilm (trade name) and INTERCEED (trade name).

After application to a surface of a tissue involved in a surgical operation, there is usually no need of suture between the anti-adhesion material and the surface of the tissue involved in the surgical operation, but if necessary, the anti-adhesion material may be sutured with the surface of tissue involved in the surgical operation.

Moreover, use of a sponge-like laminate for producing an anti-adhesion material is also provided. Detail of the use is as described hereinbefore.

In addition, a sponge-like laminate for preventing adhesions is also provided. The sponge-like laminate is specifically described hereinbefore.

### 7. Co-administered drug

Moreover, a co-administered drug, for example, an antibiotic such as streptomycin, penicillin, tobramycin, amikacin, gentamicin, neomycin or amphotericin B or an anti-inflammation drug such as aspirin, a non-steroidal analgesic antipyretic drug (NSAIDs) or acetaminophen may be administered before, simultaneously or after applying the anti-adhesion material of the present invention to a tissue involved in a surgical operation. These drugs may also be mixed with the anti-adhesion material of the present invention.
Since the sponge-like laminate is porous and has a water absorbing property, it is more adoptable for carrying a drug that can be prepared upon use, for example, as compared to non-porous Seprafilm (trade name). The sponge may be impregnated with a drug solution for administration so that adhesion prevention and topical sustained release of the drug can be realized at the same time upon administration in abdominal cavity, thoracic cavity, cardiac cavity, subarachnoid space, serous cavity (peritoneal cavity, pleural cavity, pericardial cavity), articular cavity or the like. Moreover, a drug can be carried in layers with different dissolution rates so as to allow sustained release of the drug at a faster sustained release rate and a slower sustained release rate.

The present invention will be further described in detail by way of examples, although the present invention should not be limited to these examples. Examples that are not according to the invention as defined in the claims are marked as such.

### EXAMPLES

### Example 1: Preparation of alginic acid-layered sponge (not according to the invention)

An alginic acid-layered sponge was produced as follows.

### [Reagents]

The reagents used for preparing the alginic acid-layered sponge were as follows.

Low-endotoxin sodium alginate was obtained from Mochida Pharmaceutical Co., Ltd.
·AL10: (Lot NO. 5K12202), endotoxin level 4 EU/g.
·AL500: (Lot NO. BL150713-500), endotoxin level 19 EU/g.
Calcium chloride was obtained from Wako Pure Chemical Industries, Ltd. (Product code: 036-00485).

### [Instruments used]

Untreated 35-mm dish (Product code 1000-035, IWAKI)
Micropipette (Pipetman (trade name), Gilson)
Pure water purification equipment (Elix Essential UV5 (trade name), Merck Millipore)
Freezer (SJ-56S (trade name), SHARP)
Lyophilizer (VD-550R (trade name), TAITEC)

### [Preparation procedure]

### (1) Preparation of solution

AL500 was dissolved in pure water at a concentration of 1.0 wt% to prepare an AL500 solution. Similarly, AL10 was dissolved in pure water at a concentration of 1.0 wt% to prepare an AL10 solution. Moreover, calcium chloride was dissolved in pure water to prepare 10 mM and 15 mM aqueous calcium chloride solutions, respectively.

### (2) Preparation of AL500 layer (lower layer)

1.0 mL of the AL500 solution and 1.0 mL of the 10 mM aqueous calcium chloride solution were placed into an untreated 35-mm dish using a micropipette and the resultant was homogeneously mixed by pipetting. The resultant was left to stand overnight to allow gelation. The dish was transferred to the freezer to freeze the resultant at -20°C for 4 hours.

### (3) Layering of AL10 layer (upper layer)

The dish was taken out from the freezer so as to add 1.0 mL of the AL10 solution and 1.0 mL of the 15 mM aqueous calcium chloride solution onto the frozen AL500 layer using a micropipette and the resultant was homogeneously mixed by pipetting. The dish was again placed in the freezer to freeze the resultant at -20°C for 4 hours.

### (4) Preparation of sponge

The frozen dish was placed in the lyophilizer and subjected to lyophilizing for two nights, thereby obtaining an alginic acid-layered sponge of interest.

The alginic acid-layered sponge of interest included a lower sponge-like layer (i.e., a first layer) containing AL500 and calcium chloride, and an upper sponge-like layer containing AL10 and calcium chloride (i.e., a second layer). The alginic acid-layered sponge was generally circular with a diameter of 35 mm and a thickness of 1.83 ± 0.13 cm (n = 4). The total amount of sodium alginate used in the upper layer and the lower layer was about 2.0 mg/cm². The ratio (weight ratio) of the amounts of sodium alginate used in the upper layer and the lower layer was 1:1. Furthermore, the amount of calcium chloride used was about 1.0 µmol/cm² in the upper layer and about 1.5 µmol/cm² in the lower layer.

### (5) Measurement of weight-average molecular weight

The weight-average molecular weights of the alginic acids used as the production feedstocks were measured by GPC-MALS method below.

### [Pretreatment method]

An eluent was added to dissolve a sample, and the resultant was filtrated through a 0.45 µm membrane filter to obtain a measurement solution.

### [Measurement conditions (for determination of refractive index increment (dn/dc))]

Differential refractometer: Optilab T-rEX
Measurement wavelength: 658 nm
Measurement temperature: 40°C
Solvent: 200 mM aqueous sodium nitrate solution
Sample concentration: 0.5-2.5 mg/mL (5 concentrations)

### [Measurement conditions (for determination of absolute molecular weight distribution)]

Columns: TSK gel GMPW-XL x 2 + G2500PW-XL (7.8 mm I.D. x 300 mm x 3 columns)
Eluent: 200 mM aqueous sodium nitrate solution
Flow rate: 1.0 mL/min.
Concentration: 0.05%
Detector: RI detector, light scattering detector (MALS)
Column temperature: 40°C
Injection amount: 200 µL

### [Results]

AL10: 55,000
AL500: 280,000

The sponge of a single layer containing AL10 and the sponge of a single layer containing AL500 prepared according to the procedure of steps (1), (2) and (4) above were subjected to electron-beam sterilization, and then dissolved in an EDTA (ethylenediaminetetraacetic acid) solution to respectively measure their molecular weights by GPC-MALS method. The results are shown below.

### [Results]

(Where the radiation dose for electron-beam sterilization was 20 kGy)
AL10: 36,000
AL500: 75,000
(Where the radiation dose for electron-beam sterilization was 40 kGy)
AL10: 27,000
AL500: 45,000

In Examples 3, 3-2, 4 and 9 described below, the layered sponges used were those that had been subjected to electron-beam sterilization (20 kGy).

In Examples 6 and 7 described below, the layered sponges used were not sterilized.

### Example 1-2: Preparation of alginic acid-layered sponge

### (1) Preparation of sponge

Using the alginic acids listed in [Reagents] below, i.e., AL100 or AL500 as a feedstock of the lower layer and AL10 or AL20 as a feedstock of the upper layer, alginic acid-layered sponges were prepared in combinations of AL10 (upper layer)-AL100 (lower layer), AL20 (upper layer)-AL100 (lower layer) and AL20 (upper layer)-AL500 (lower layer), respectively, according to the method described in Example 1.

Herein, a combination of an upper layer and a lower layer may also be referred to as "lower layer/upper layer" for the sake of convenience. For example, according to this representation, a combination of AL10 (upper layer)-AL100 (lower layer) may be referred to as "AL100/AL10", and a combination of AL20 (upper layer)-AL500 (lower layer) may be referred to as "AL500/AL20".

### [Reagents]

·AL10: Same as Example 1
·AL20: (Lot NO. BL150713-20), endotoxin level 13 EU/g
·AL100: (Lot NO. 5G17201), endotoxin level 6 EU/g
·AL500: Same as Example 1

### (2) Measurement of weight-average molecular weights

Furthermore, the weight-average molecular weights of AL20 and AL100 among the alginic acids used for sponge preparation were measured by GPC-MALS method according to the method described in Example 1.

### [Results]

AL20: 82,000
AL100: 170,000

The molecular weights of the sponge of a single layer containing AL20 and the sponge of a single layer containing AL100 which were prepared according to the method described in Example 1 were also measured after the electron-beam sterilization according to the method described in Example 1. The results are shown below.

### [Results]

(Where the radiation dose for electron-beam sterilization was 20 kGy)
AL20: 46,000
AL100: 63,000
(Where the radiation dose for electron-beam sterilization was 40 kGy)
AL20: 33,000
AL100: 40,000

### Example 1-3: Preparation of alginic acid-layered sponge (curing with calcium gluconate)

An alginic acid-layered sponge was prepared according to the method of Example 1 except that 10 mM calcium gluconate (from Wako Pure Chemical Industries, Ltd.) was used instead of 10 mM calcium chloride upon gelation of the AL500 layer. The prepared sponge was used in Experimental group 1 of Example 3-3 below.

### Example 1-4: Preparation of alginic acid-layered sponge (EOG sterilization)

An alginic acid-layered sponge, AL10 (upper layer)-AL100 (lower layer), was prepared according to the procedure of steps (1)-(4) in Example 1 except that AL100 used in Example 1-2 was used as an alginic acid of the lower layer. The resulting sponge was sterilized using ethylene oxide gas according to a common method. The resulting sponge was used in Experimental group 4 of Example 3-3 below.

### Example 1-5: Preparation of alginic acid-layered sponge (pressing after 1-step freezing)

A solution was prepared according to the procedure of step (1) in Example 1 except that AL100 used in Example 1-2 was used as an alginic acid of the lower layer, to further carry out the procedure of step (2) and the subsequent steps to prepare an alginic acid-layered sponge.

### (2) Preparation of AL100 layer (lower layer)

1.0 mL of the AL100 solution and 1.0 mL of the 10 mM aqueous calcium chloride solution were placed into an untreated 35-mm dish using a micropipette and the resultant was homogeneously mixed by pipetting. The resultant was left to stand for 1-2 hours to allow gelation.

### (3) Layering of AL10 layer (upper layer)

1.0 mL of the AL10 solution and 1.0 mL of the 15 mM aqueous calcium chloride solution were added onto the gelated AL100 layer using a micropipette and the resultant was homogeneously mixed by pipetting. The dish was transferred to the freezer to freeze the resultant at -20°C for 4 hours.

### (4) Preparation of sponge

The frozen dish was placed in the lyophilizer and subjected to lyophilizing for two nights, thereby obtaining an alginic acid-layered sponge of interest.

### (5) Pressing of sponge

The sponge obtained in (4) above was set in a press machine (from AS ONE Corporation, product name AH-1T). The sponge was pressed at a pressure of 10 MPa at room temperature and held for 5 minutes. The pressed sponge was sterilized with an electron beam according to a common method. The resulting sponge was used in Experimental group 5 of Example 3-3 below.

### Example 2: Determination of dissolution rates of respective layers of alginic acid-layered sponge (Immersing approach)

Layered sponges that have either the upper or lower layer fluorescently modified were prepared to determine their dissolution rates. Detail of the procedure will be described below. Here, the alginic acid was labeled by a common method using FTSC (fluorescein-5-thiosemicarbazide) as a fluorescent labeling reagent.

The fluorescently-labeled alginic acid was used to prepare a layered sponge according to the method described in Example 1.

### [Materials]

The low-endotoxin sodium alginate was as described in Example 1. A phosphate buffer solution was prepared using sodium dihydrogen phosphate (197-09705 (trade name), Wako Pure Chemical Industries, Ltd.), potassium dihydrogen phosphate (166-04255 (trade name), Wako Pure Chemical Industries, Ltd.), sodium chloride (191-01665 (trade name), Wako Pure Chemical Industries, Ltd.), and potassium chloride (166-17945 (trade name), Wako Pure Chemical Industries, Ltd.). Ethylenediamine tetraacetic acid sodium (N001) was purchased from Dojindo.

### [Instruments used]

8 mm-diameter biopsy punch (BP-80F (trade name), Kai medical)
96-well black microplate (137101 (trade name), Nunc)
Fluorescent plate reader (ARVO X3 (trade name), Perkin Elmer)

### [Procedure]

First, the fluorescently modified layered sponge was punched out with the 8 mm-diameter biopsy punch (BP-60F (trade name), Kai medical). The resultant was immersed in 10 mL of a 150 mM phosphate buffer solution (pH 7.5), and 200 µL each of the immersion solution was collected at regular intervals. The collected solutions were transferred to the 96-well microplate, and the fluorescent intensities were determined with the fluorescence microplate reader to quantify the amounts of the dissolved alginic acid.

### [Results]

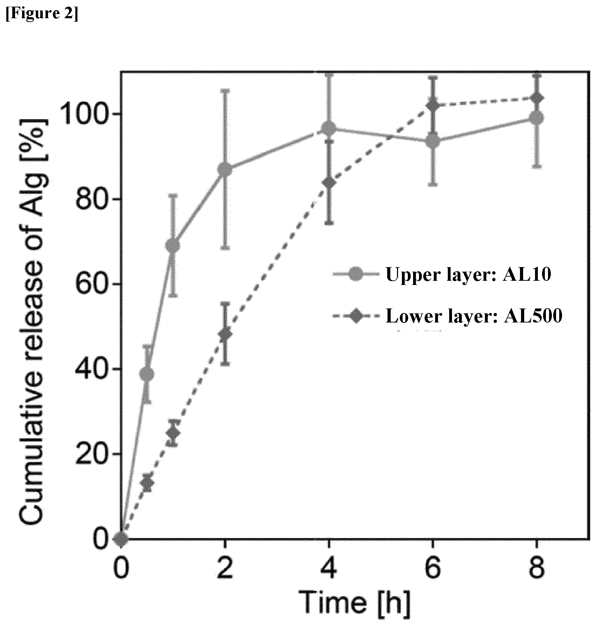

The results from the determination of the dissolving behavior of the respective layers of the layered sponge are shown in Figure 2. As can be appreciated from Figure 2, for the lower layer, 25 ± 10 wt% of the monovalent metal salt of alginic acid was eluted within an hour, and 80 ± 10 wt% was eluted within 4 hours. Meanwhile, for the upper layer, 70 ± 10 wt% of the monovalent metal salt of alginic acid was eluted within an hour and 90 ± 10 wt% was eluted within 4 hours. Moreover, the proportion of the amount of the monovalent metal salt of alginic acid eluted from the lower layer was 36% (less than 50%) after an hour and 55% (less than 70%) after 2 hours following the start of the measurement, when taking the amount of the monovalent metal salt of alginic acid eluted from the upper layer as a base of 100%.

Thus, the dissolution rate of the upper layer was confirmed to be faster than that of the lower layer. For example, if the alginic acid sponge is applied to a wound of an organ in the abdominal cavity such that the lower layer faces the wound while the upper layer faces the abdominal cavity, the lower layer is considered to remain on the wound and prevent an adhesion of the wound while the upper layer is considered to dissolve relatively faster and suppress *de novo* adhesions such as adhesions in the whole abdominal cavity that are often formed remote from the wound.

Dissolution rates were similarly determined for the respective layered sponges produced in Example 1-2 (AL10 (upper layer)-AL100 (lower layer); AL20 (upper layer)-AL100 (lower layer); and AL20 (upper layer)-AL500 (lower layer)). As a result, they showed similar dissolving behavior (the dissolution rate of the upper layer being faster than that of the lower layer) as that of the layered sponge produced in Example 1 (AL10 (upper layer)-AL500 (lower layer)).

### Example 2-2: Determination of dissolution rates of respective layers of alginic acid-layered sponge (Applying approach)

AL500/AL10 layered sponges that have either the upper or lower layer fluorescently modified were prepared according to the method described in Example 2 to determine the dissolution rates by the following procedure.

### [Procedure]

1) A fluorescently modified layered sponge was punched out with the 8 mm-diameter biopsy punch (BP-60F (trade name), Kai medical).
2) Agarose (Product code: 010-08725, Wako Pure Chemical Industries, Ltd.) was dissolved in hot water to 2 wt% and cooled to room temperature to prepare an agarose gel. The agarose gel was cut into a 2 cm x 2 cm square, which was immersed and wetted with a phosphate buffer solution in a glass petri dish. A phosphate buffer solution (pH 7.5) was added to the glass petri dish such that the liquid level is slightly beneath the upper surface of the agarose gel.
3) The sponge obtained in 1) was left to stand on the agarose gel, and the phosphate buffer solution was collected at regular intervals to determine the fluorescent intensities.

### [Results]

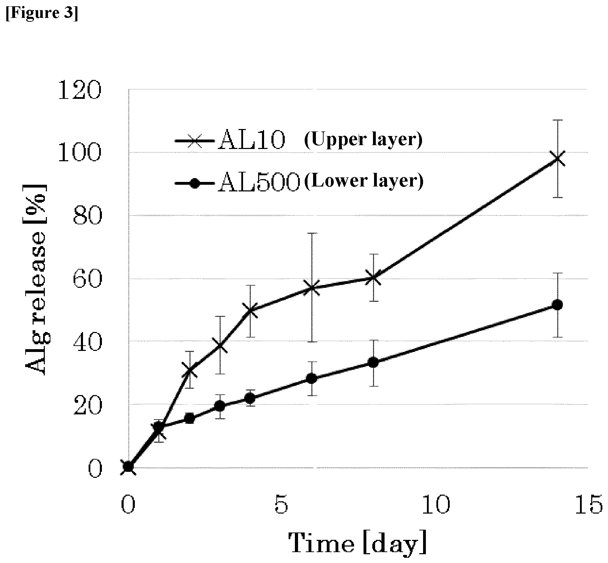

The results from the determination of the dissolving behavior of the respectively layers of the layered sponge are shown in Table 1 and Figure 3. Each of the values represents wt% of the eluted alginic acid at each time point, when taking the weight of the alginic acid prior to the test as a base of 100 wt%.

**[Table 1]**

| Time (day) | AL10 (Upper layer) | | AL500 (Lower layer) | |
|---|---|---|---|---|
| | Average | Standard error | Average | Standard error |
| 0 | 0.129 | 0.0249 | 0.251 | 0.0863 |
| 1 | 11.347 | 3.1383 | 13.053 | 2.1654 |
| 2 | 30.988 | 5.8034 | 15.682 | 1.6327 |
| 3 | 38.743 | 9.1778 | 19.466 | 3.7604 |
| 4 | 49.679 | 8.2186 | 22.032 | 2.5436 |
| 6 | 57.023 | 17.157 | 28.135 | 5.4421 |
| 8 | 60.233 | 7.4783 | 33.233 | 7.3126 |
| 14 | 97.869 | 12.305 | 51.506 | 10.031 |

In the test system of this method, moisture is supplied only from the attaching surface and thus the dissolving behavior of the layered sponge can be confirmed in an environment more closely resembling a living body. In this test system, the alginic acid in the upper layer reaches the agarose gel via the side surface and the lower layer and is then eluted into the phosphate buffer solution in the petri dish. As can be appreciated from Table 1 and Figure 3, about 16 ± 2 wt% of the monovalent metal salt of alginic acid in the lower layer is eluted within 2 days, and about 33 ± 7 wt% is eluted within 8 days. Meanwhile, about 31 ± 6 wt% of the monovalent metal salt of alginic acid in the upper layer is eluted within 2 days, and about 60 ± 7 wt% is eluted within 8 days. Meanwhile, the proportion of the amount of the monovalent metal salt of alginic acid eluted from the lower layer was about 50 ± 5% (about 44% to about 55%) 2 days or later following the start of the measurement on, when taking the amount of the monovalent metal salt of alginic acid eluted from the upper layer as a base of 100%. Hence, the dissolution rate of the upper layer was confirmed to be faster than that of the lower layer even under conditions more closely resembling those in a living body.

### Example 3: Partially resected rat hepatic model

Partially resected rat hepatic models were used to evaluate formation of adhesions. A partially resected rat hepatic model is a model that generates serious inflammation and that allows highly reproducible observation of highly intense adhesion formation (Shimizu A et al., (2014) Surg Today. (44): 314-323). Specifically, formation of an adhesion was evaluated as follows.

### [Materials]

The low-endotoxin sodium alginates were the same as described in Example 1.

Seprafilm (trade name) was a sheet-like material of a mixture of carboxymethyl cellulose (CMC) and hyaluronic acid, which was obtained from Genzyme GmbH.

Interceed (trade name) was an oxidized regenerated cellulose sheet, which was obtained from Johnson & Johnson.

### [Experimental groups]

Control group (n = 8): 3 cm of the margin of the left lateral lobe was measured and dissected, and bleeding was stopped by coagulation (untreated control group).

AL500/AL10 layered sponge group (n = 8): The alginic acid sponge produced in Example 1 was adopted as an anti-adhesion material.

Seprafilm group (n = 8): 2 x 3 cm Seprafilm was adopted as an anti-adhesion material.

Interceed group (n = 8): 2 x 3 cm Interceed was adopted as an anti-adhesion material.

### [Procedure]

The rat was anesthetized by intraperitoneally administering 35 mg/kg of pentobarbital thereto. The weight was measured using an electronic balance. Subsequently, midline abdominal incision was made in the rat. Then, the abdominal wall was pulled up with forceps to cut the abdominal wall. As preparation prior to hepatic resection, the left lateral lobe was pulled out from inside the abdominal cavity and gauze was laid underneath. Thereafter, actual hepatic resection was carried out. Specifically, a ruler was applied to the liver to find out the position for obtaining a 3-cm resected surface, which was marked by cauterizing both ends with a bipolar. A linear cut was made between the marked two points. For the control group, the abdomen was closed immediately thereafter to complete the treatment. For the groups to be applied with the anti-adhesion material, the anti-adhesion material was applied after removing the gauze. Subsequently, sutures were made in two steps in the abdominal wall and the skin to close the abdomen. The abdominal wall was sutured using a biodegradable suture while the skin was sutured with a nonabsorbable suture. A week following the abdominal closure, the rat was euthanized by administering about 2 mL of pentobarbital as an excessive dose of anesthesia and the weight was measured using an electronic balance. Thereafter, the abdomen was reopened to evaluate adhesions as follows. After dissecting the spleen from the abdominal cavity, the spleen weight was measured using an electronic balance.

### [Evaluation of adhesions]

The adhesions were evaluated as follows.

### (1) Resected surface

The following evaluations (a) and (b) were made on the resected liver surface described in [Procedure] above.

### (a) Grades of adhesion

The adhesion was evaluated by visual observation. The adhesion of the resected liver surface was scored based on the following scoring.

### Scoring of adhesion:

Grade 0: No adhesion is observed at all
Grade 1: Adhesion of a degree that allows separation with gravity (physiological dissection)
Grade 2: Adhesion that can be separated with forceps (blunt dissection)
Grade 3: Adhesion that cannot be separated without scissors or a scalpel (sharp dissection)

### (b) Extent of adhesion

The width of the adhesion formed along the 3 cm-long resected liver surface was measured with a ruler and expressed in length (unit: mm) (thus, the maximum extent of the resected surface would be 30 mm).

### (2) Unresected surface

The following evaluations (a) and (b) were made on parts other than the resected liver surface, specifically, liver surface, greater omentum, peritoneal, small intestine, a part right beneath the midline wound and the like. Adhesions on the unresected surface serve as an indicator of *de novo* adhesions.

### (a) Grades of adhesion

The adhesion was evaluated by visual observation. The adhesion of the part other than the resected liver surface was scored based on the following scoring. The part of the adhesion was not specified, and the maximum adhesion score observed was recorded as the adhesion score of the test animal.

### Scoring of adhesion:

Grade 0: No adhesion is observed at all
Grade 1: Adhesion that can be separated with gravity (physiological dissection)
Grade 2: Adhesion that can be separated with forceps (blunt dissection)
Grade 3: Adhesion that cannot be separated without scissors or a scalpel (sharp dissection)

### (b) Extent of adhesion

The width of the tissue site forming an adhesion on the part other than the resected liver surface was measured with a ruler and expressed in length (unit: mm). Similar to (2)(a) above, the part of the adhesion was not specified, and the maximum width of the adhesion observed was recorded as the adhesion extent of the test animal.

### [Results]

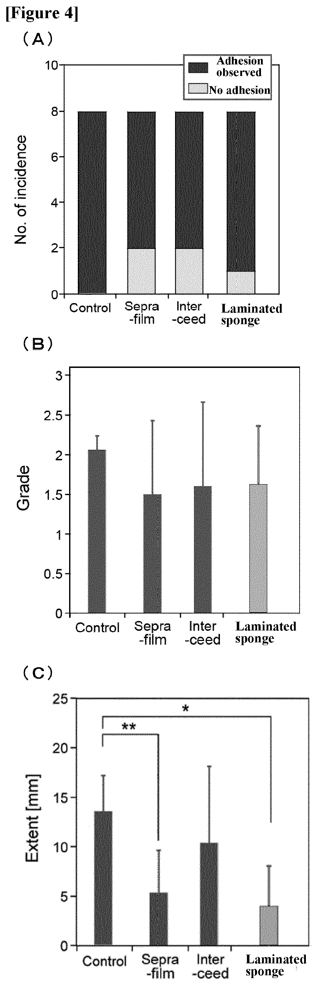

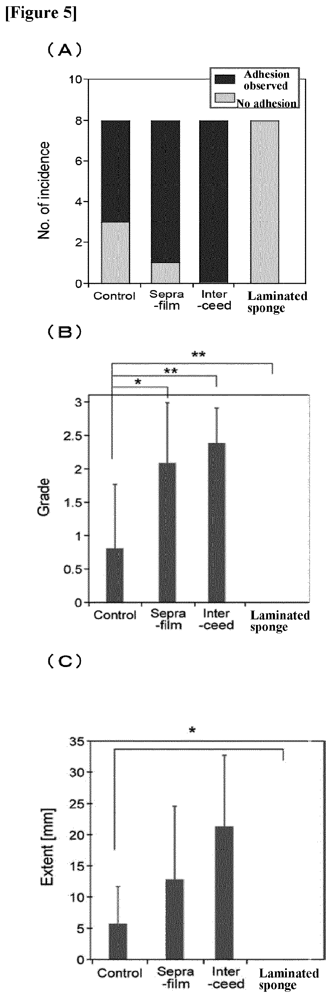

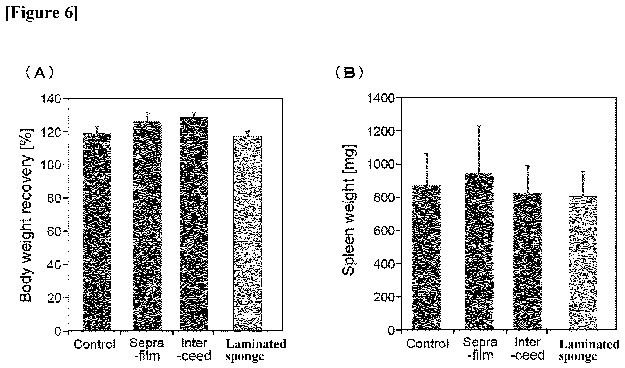

The results from the adhesion evaluations are shown in Figure 4 (resected surface) and Figure 5 (unresected surface). In addition, the results from the weight measurements and spleen weight measurements are shown in Figure 6.

For each group, the adhesions on the resected surfaces were found to be suppressed as compared to the control group (Figures 4(A)-(C)).

A remarkable adhesion prevention effect was confirmed on the unresected surfaces for the AL500/AL10 layered sponge group (Figures 5(A)-(C)). Seprafilm (trade name) and Interceed (trade name) that were used as the positive controls had no adhesion prevention effect, and the adhesions were found to worsen as compared to the control group. Meanwhile, a remarkable adhesion prevention effect was confirmed in the AL500/AL10 layered sponge group.

As to the weight and the spleen weight, there was no significant difference among the control group, the Seprafilm group, the Interceed group and the AL500/AL10 layered sponge group, confirming that the application of the AL500/AL10 layered sponge had no adverse effect on the living body (Figures 6(A) and (B)).

Herein, unless otherwise specified, the significance tests in the examples were conducted by Student's t-test, except that evaluations by grades were conducted by Mann-Whitney U test.

### Example 3-2: Rat hepatic model partially resected with Pean clamp

The adhesion grades and the adhesion extents of resected and unresected liver surfaces were evaluated using the same material, experimental groups, procedures and adhesion evaluation methods as Example 3 except that a Pean clamp was used upon liver dissection.

Dissection of the liver using a Pean clamp was carried out specifically as follows. Specifically, the "linear cut between the marked two points" described in [Procedure] of Example 3 was made by crushing liver parenchyma with the Pean clamp, and cauterizing the exposed blood vessel with a bipolar.

### [Results]

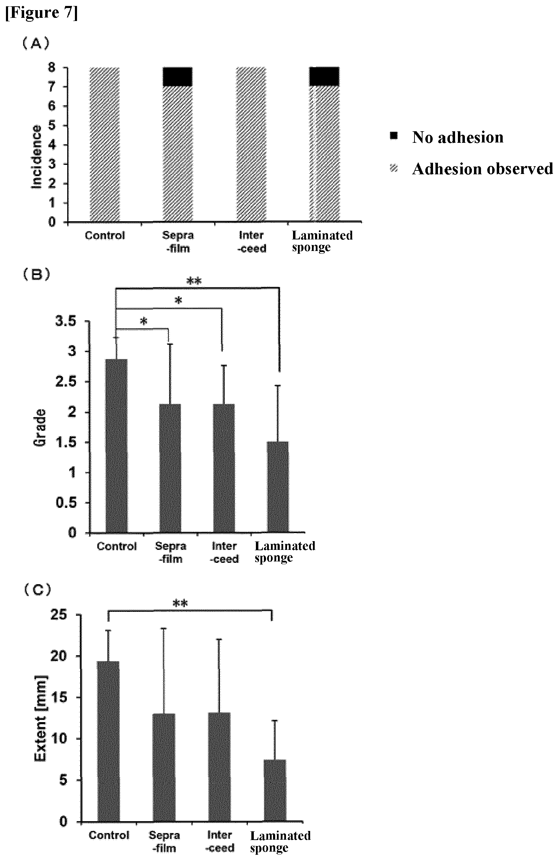

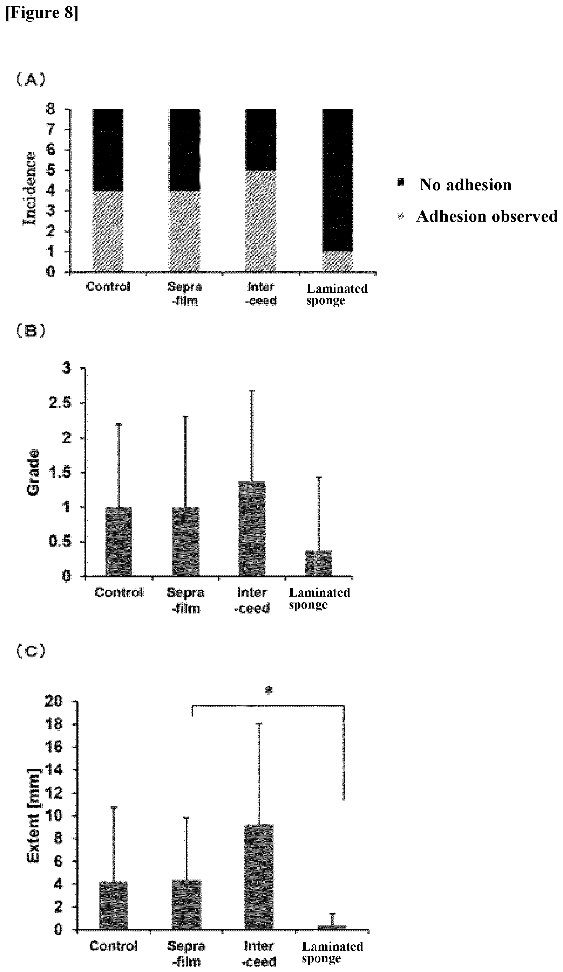

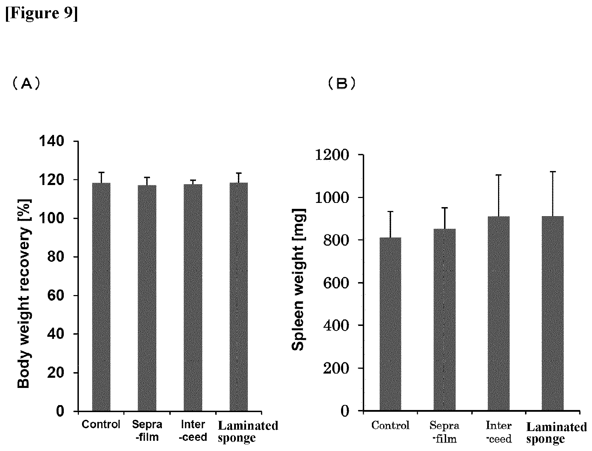

The results from the adhesion evaluations are shown in Figure 7 (resected surface) and Figure 8 (unresected surface). In addition, the results from the weight measurements and spleen weight measurements are shown in Figure 9.

For each group (n = 8), the adhesions on the resected surfaces were found to be suppressed as compared to the control group (n = 8), where the AL500/AL10 layered sponge group showed a statistically significant difference from the control group (Figures 7(A)-(C)).

A remarkable adhesion prevention effect was confirmed on the unresected surfaces of the AL500/AL10 layered sponge group (Figures 8(A)-(C)). No adhesion prevention effect was observed with Seprafilm (trade name) that was used as the positive control while the adhesion tended to worsen with Interceed (trade name) as compared to the control group. Meanwhile, a remarkable adhesion prevention effect was confirmed for the AL500/AL10 layered sponge group.

As to the weights and the spleen weights, there was no significant difference among the control group, the Seprafilm group, the Interceed group and the AL500/AL10 layered sponge group, confirming that the application of the AL500/AL10 layered sponge had no adverse effect on the living body (Figures 9(A) and (B)).

### Example 3-3: Rat hepatic model partially resected with Pean clamp

The adhesion grades and the adhesion extents of resected and unresected liver surfaces were evaluated for the following experimental groups employing the method of Example 3-2.
Experimental group 1: AL500/AL10, cured with calcium gluconate [prepared in Example 1-3]
Experimental group 2: AL500/AL10, pressed [prepared in Example 6 (1-2)]
Experimental group 3: AL100/AL10, sterilized with an electron beam [prepared in Example 1-2]
Experimental group 4: AL100/AL10, sterilized with EOG [prepared in Example 1-4]
Experimental group 5: AL100/AL10, 1-step frozen, pressed [prepared in Example 1-5]

Here, the untreated control group, the Seprafilm group and the AL500/AL10 [prepared in Example 1] were separately evaluated to compare their results with the results of the above-mentioned experimental groups.

### [Results]

All of Experimental groups 1-5 had the same effect as the layered sponge prepared in Example 1.

### Example 4: Visualization of respective layers of the alginic acid-layered sponge by fluorescent labeling

The respective layers of the alginic acid-layered sponge were visually examined by fluorescent labeling as follows.

### [Materials]

Low-endotoxin sodium alginates were the same as described in Example 1.

### [Instruments used]

Handheld UV lamp (UVGL-58 (trade name), UVP)

### [Procedure]

The procedure for resecting a rat liver was the same as described in Example 3. An alginic acid sponge having either the first or second layer fluorescently labeled was placed on the resected surface of the prepared liver. In order to facilitate observation of the remaining level of the material, a layered sponge using an alginic acid for 4.0 mg/cm² which was greater than Example 1 was prepared and used according to the method of Example 1. Thereafter, the abdomen was closed according to the procedure described in Example 3, and the abdomen was reopened a week later. An ultraviolet lamp was used to irradiate inside the exposed abdominal cavity so as to visualize the distribution of the fluorescently-labeled alginic acid inside the abdominal cavity.

As a result, the AL10 layer was confirmed to be widely distributed over the peritoneal surface as well as the resected surface. From this, the second layer of the sponge-like laminate was suggested to rapidly melt and spread inside the abdominal cavity owing to the relatively low weight-average molecular weight of the monovalent metal salt of alginic acid.

Meanwhile, fluorescence from the AL500 layer was partially observed on the abdominal wall and more significantly observed on the resected surface. From this, the first layer of the sponge-like laminate was suggested to remain on the resected surface and serve as a physical barrier owing to the relatively high weight-average molecular weight of the monovalent metal salt of alginic acid.

### Example 5: Wrapping test

In order to see the adhesive followability of the alginic acid-layered sponge on a curved surface, a wrapping test was conducted as follows.

### [Materials]

A low-endotoxin sodium alginate was the same as described in Example 1. Agar (010-08725) was purchased from Wako Pure Chemical Industries, Ltd.

### [Procedure]

Agar was dissolved in hot water and then poured and cooled in a columnar mold to give an agarose gel column with a diameter of 20 mm. This was used as a tubular organ model, around which the sponge produced in Example 1 was wrapped to verify the wrapping followability.

As a result, the sponge was confirmed to be capable of being wrapped around the column resembling an intestinal tract owing to flexibility of the alginic acid-layered sponge. This suggested that an anti-adhesion material comprising a sponge-like laminate can also be used for intestinal anastomosis and the like.

### Example 6: Pressing of sponge and swelling test thereof

Pressing of the alginic acid-layered sponge, and measurement of the thickness thereof and swelling test after the pressing were conducted as follows.

### [Materials]

The alginic acid-layered sponge (AL10 (upper layer)-AL500 (lower layer)) was the same as described in Example 1.

### [Procedure]

### (1) Pressing and measurement of thickness after pressing

### (1-1) Manual pressing

The alginic acid-layered sponge was placed on a flat surface and pressed with the palm via an acrylic ruler such that the whole sponge was uniformly pressed. The thickness of the sponge was measured with an electronic caliper before and after the pressing. The average was calculated (n = 4).

### (1-2) Pressing with press machine

The alginic acid-layered sponge was set on a press machine (product name AH-1T from AS ONE Corporation). The alginic acid-layered sponge was pressed at a pressure of 10 MPa at room temperature and held for 5 minutes. The thickness of the sponge was measured with an electronic caliper before and after the pressing. The average was calculated (n = 4).

### (2) Swelling test

An agarose gel was prepared in a glass petri dish in the same manner as Example 2-2. The agarose gel was wetted with pure water. The alginic acid-layered sponges before and after pressing were cut into a 1 cm x 1 cm square and placed on the agarose gel. The thickness of the sponge was calculated by transversely taking pictures at regular intervals so as to confirm the presence of influence of pressing on swelling (before pressing: n = 3; after pressing: n = 3). For the swelling test, an alginic acid-layered sponge pressed with the press machine was used as the pressed alginic acid-layered sponge.

### [Results]

The results from the thickness measurement after pressing are showing in Table 2.

**[Table 2]**

| | Before pressing | After manual presssing | After pressing with press machine |
|---|---|---|---|
| Average thickness (mm) | 1.5 | 0.33 | 0.16 |

The average thickness of the sponges was about 1.5 mm before pressing, about 0.33 mm after manual pressing and about 0.16 mm after pressing with the press machine. The thickness of the pressed sponge did not increase with time and the above-mentioned thickness was maintained.

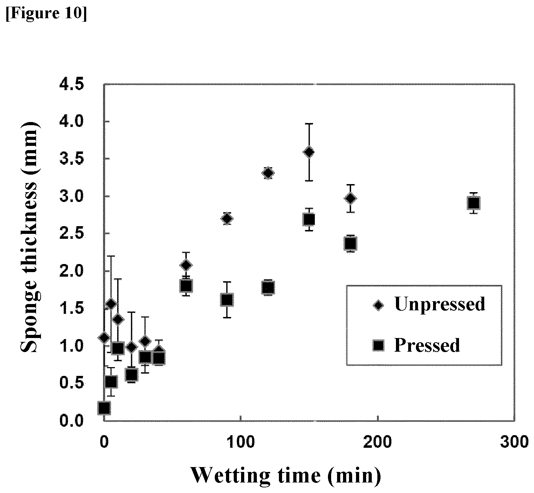

Changes in the thickness of the sponge with time in the swelling test are shown in Figure 10. Regarding the results from the swelling test, the pressed alginic acid-layered sponge was confirmed to restore the thickness substantially equal to that of the unpressed alginic acid sponge by water absorption.

This suggested that since the sponge can be made compact by pressing, the sponge that serves as an anti-adhesion material can be applied relatively easily to an affected area via a trocar or the like upon an endoscopic surgery.

It was also suggested that the pressed sponge applied to the affected area absorbs moisture present in or applied to the affected area to restore the thickness. By restoring the thickness, the layered sponge can exert its functions.

### Example 7: Spraying test

Vulnerability of the alginic acid-layered sponge and Seprafilm (trade name) upon water absorption were evaluated by the following test.

### [Materials]

The alginic acid-layered sponge was the same as that described in Example 1 and Seprafilm (trade name) was the same as that described in Example 3. Moreover, the pressed alginic acid-layered sponge used was one that was pressed with the press machine described in Example 6.

### [Procedure]

1 cm x 2 cm test pieces were made from the alginic acid-layered sponge and Seprafilm (trade name). A double-sided tape was adhered on one end (1 cm x 1 cm) of the test piece, and held at an edge of a test board so that the test piece was fixed with the other end (1 cm x 1 cm) sticking out in the air.

Pure water was sprayed five times on each of the test pieces using an atomizer. Video was taken to record the course of the test piece bending downward due to wetting.

Based on an image analysis of the obtained video, both the height and the angle of the tip of the test piece with respect to the test board were calculated and their changes were plotted with time.

### [Results]

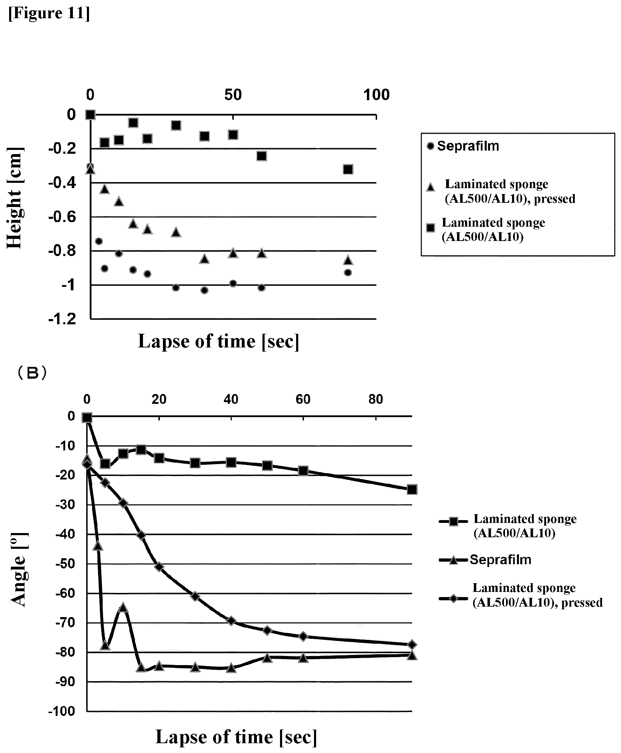

The results are shown in Figure 11.

The decrease in the height of the alginic acid-layered sponge was within 2 mm until 50 seconds following spraying, and about 3 mm after 90 seconds (Figure 11(A)). The decrease in the height of the pressed alginic acid-layered sponge was also about 9 mm after 90 seconds following spraying. On the other hand, Seprafilm (trade name) showed a significant decrease in the height immediately after spraying (Figure 11(A)). The results for the angle was similar to that for the height (Figure 11(B)).

This suggested that the alginic acid-layered sponge can maintain its shape and strength for a while in a water absorbed state either pressed or unpressed. Therefore, it has advantages such as that it can be reapplied to adjust its applied position when applied to an affected area as an anti-adhesion material, or that it can avoid situation like it cannot be smoothly opened by absorbing moisture in a trocar or the like when the sponge is applied as an anti-adhesion material to an affected area via the trocar or the like upon an endoscopic surgery. The alginic acid-layered sponge has been confirmed to have a favorable pressure bonding property to an affected area or to a model system thereof in Examples 3, 5 and else.

### Example 8: Young's modulus/strength at break

Measurement was carried out with a tensile tester to evaluate the mechanical characteristics of the alginic acid-layered sponge.

### [Materials]

The alginic acid-layered sponge prepared in Example 1 and the alginic acid-layered sponge prepared and pressed with a press machine in Example 6 were used as an unpressed alginic acid-layered sponge and a pressed alginic acid-layered sponge, respectively.

### [Procedure]

JIS K6251 Type 8 dumbbell-shaped tensile pieces (width 10 mm) were prepared from the respective sponges. Each of the pieces was subjected to a tensile tester (CR-3000EX-S from Sun Scientific Co., Ltd.) to acquire a stress-strain curve. The Young's modulus and the strength at break were determined based on the acquired stress-strain curve.

### [Results]

The measurement results were as follows (average ± standard error).

Young's modulus:
AL500/AL10 sponge (unpressed) 0.79 ± 0.164 MPa
AL500/AL10 sponge (pressed) 14.88 ± 1.434 MPa

Strength at break:
AL500/AL10 sponge (unpressed) 51.55 ± 6.391 kPa
AL500/AL10 sponge (pressed) 272.80 ± 61.892 kPa

As can be appreciated from the above results, the Young's modulus and the strength at break increased and the sponge was toughened by pressing the sponge.

### Example 8-1: Young's modulus/strength at break (2)

### [Material]

Alginic acid-layered sponges having the following compositions were prepared.

**[Table 3]**

| Preparation | First layer (lower layer) | | | Second layer (upper layer) | | | Filled amount | Process |
|---|---|---|---|---|---|---|---|---|
| 1 | AL20 | + | CaCl₂ 15 mM | AL10 | + | CaCl₂ 15 mM | 16+16 mL | 2-step freezing |
| 2 | AL500 | + | Ca-Glu 10 mM | AL20 | + | Ca-Glu 15 mM | 16+16 mL | 2-step freezing |
| 3 | AL100 | + | Ca-Glu 10 mM | AL20 | + | Ca-Glu 15 mM | 16+16 mL | 2-step freezing |
| 4 | AL 500: AL100 =2:1 | + | Ca-Glu 10 mM | AL10 | + | Glu-15 mM | 16+16 mL | 2-step freezing |
| 5 | AL 500: AL100 =1:1 | + | Ca-Glu 10 mM | AL10 | + | Ca-Glu 15 mM | 16+16 mL | 2-step freezing |
| 6 | AL 500: AL100 =1:2 | + | Ca-Glu 10 mM | AL10 | + | Ca-Glu 15 mM | 16+16 mL | 2-step freezing |
| 7 | AL100 | + | Ca-Glu 10 mM | AL10 | + | Ca-Glu 15 mM | 16+16 mL | 1-step freezing |
| 8 | AL100 | + | Ca-Glu 10 mM | AL10 | + | Ca-Glu 15 mM | 16+16 mL | 2-step freezing |

| | | | | | | | | |
|---|---|---|---|---|---|---|---|---|
| *"Ca-Glu" represents calcium gluconate. | | | | | | | | |

Preparation 1 was made by preparing an alginic acid-layered sponge according to the method described in Example 1, and pressing the resultant according to the method described in Example 6 (1-2). Preparations 2 and 3 were made by preparing alginic acid-layered sponges according to the method described in Example 1-3, and pressing the resultants according to the method described in Example 6 (1-2). Preparations 4 to 6 were made by preparing alginic acid-layered sponges according to the method described in Example 1 except that a mixture of AL500 (same as Example 1) and AL100 (same as Example 1-2) at a predetermined weight ratio was used as the lower sodium alginate layer, and pressing the resultant according to the method described in Example 6 (1-2). Preparation 7 was made according to the method described in Example 1-5. Preparation 8 was made according to the method described in Example 1. Some of Preparations 7 and 8 were pressed according to the method described in Example 6 (1-2).

### [Procedure]

Young's modulus and strength at break of each preparation were determined according to the method described in Example 8 (n = 4).

### [Results]

The measurement results were as follows. All of the prepared alginic acid-layered sponges had strength and flexibility suitable as an anti-adhesion material.

**[Table 4]**

| Preparation | Strength at break (kPa) | | Young's modulus (MPa) | | Strength at break/ Young's modulus ratio | Thickness (mm) |
|---|---|---|---|---|---|---|
| | Average | S.D. | Average | S.D. | Average | Average |
| 1 | 382.01 | 126.55 | 28.27 | 12.48 | 13.51 | 0.114 |
| 2 | 104.94 | 31.88 | 3.60 | 2.58 | 29.11 | 0.107 |
| 3 | 139.22 | 66.66 | 8.55 | 3.48 | 16.28 | 0.138 |
| 4 | 208.17 | 73.63 | 14.05 | 4.34 | 14.82 | 0.118 |
| 5 | 217.72 | 129.53 | 9.95 | 6.92 | 21.89 | 0.113 |
| 6 | 239.51 | 173.40 | 19.40 | 9.24 | 12.35 | 0.118 |
| 7 | 2386.83 | 454.86 | 162.12 | 36.92 | 14.72 | 0.100 |
| 8 | 1911.27 | 209.89 | 81.51 | 15.01 | 23.45 | 0.114 |
| 7 (unpressed) | 26.03 | 9.76 | 1.26 | 0.48 | 20.74 | 2.095 |
| 8 (unpressed) | 16.25 | 4.19 | 0.48 | 0.14 | 33.72 | 2.474 |

### Example 9: Water sorption test

The following test was conducted to evaluate the water absorbing property of the alginic acid-layered sponges.

### [Materials]

Agarose gel/glass petri dish were the same as Example 2-2.

AL500/AL10 (Example 1), AL100/AL10 (Example 1-2) and AL500G/AL10 (Example 1-3) sponges were targeted for the measurement. All of the sponges targeted for the measurement were sterilized with an electron beam (20 kGy). Each sponge was punched out with the 8 mm-diameter biopsy punch (BP-60F (trade name), Kai medical) to be used for the test.

### [Procedure]

A mesh was placed stably on the agarose gel in the petri dish filled with phosphate buffered saline, on which the sponge targeted for the measurement was further placed to determine the changes in the sponge weight (%) for 6 hours.

### [Results]

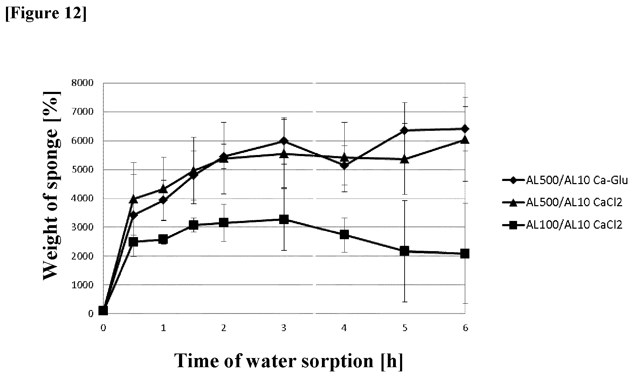

The measurement results are shown in Figure 12. All of the sponges showed good water absorbing property. Here, the AL100/AL10 sponge had a weight increase lower than other sponges because water sorption and sponge dissolution proceeded simultaneously.

### DESCRIPTION OF REFERENCE NUMERALS

- 1: Anti-adhesion material
- 2: First layer
- 3: Second layer
- 4: Sponge-like laminate

## Claims

1. An anti-adhesion material comprising a biocompatible sponge-like laminate that is porous, includes first and second sponge-like layers containing low-endotoxin monovalent metal salts of alginic acid which are at least partially crosslinked with a curing agent, and is obtainable by commonly or individually lyophilizing the cured first and second layers of the laminate, wherein a weight-average molecular weight of the monovalent metal salt of alginic acid in the first layer is 30,000-300,000, a weight-average molecular weight of the monovalent metal salt of alginic acid in the second layer is 1,000-200,000, said low-endotoxin monovalent metal salts of alginic acid of the first and second layer have an endotoxin content of 500 Endotoxin Units (EU)/g or less measured using a limulus reagent, the weight-average molecular weights are measured by GPC-MALS method following a decrosslinking treatment, and the weight-average molecular weight of the monovalent metal salt of alginic acid in the first layer is higher than the weight-average molecular weight of the monovalent metal salt of alginic acid in the second layer; said anti-adhesion material having one or more of the following characteristics:
(1) the Young's modulus determined based on a stress-strain curve acquired by subjecting a JIS K6251 Type 8-shaped tensile test piece to a tensile tester is 0.3-300 MPa;
(2) the strength at break determined based on the stress-strain curve acquired by subjecting the JIS K6251 Type 8-shaped tensile test piece to the tensile tester is 5-5000 kPa; and
(3) the ratio of the strength at break to the Young's modulus of the sponge-like laminate determined based on a stress-strain curve acquired by subjecting a JIS K6251 Type 8-shaped tensile test piece to a tensile tester is 2-50.

2. The anti-adhesion material according to claim 1, wherein the total amount of the low-endotoxin monovalent metal salts of alginic acid used in the first layer and the second layer is in a range of 0.1 mg/cm²-3 mg/cm².

3. The anti-adhesion material according to either one of claims 1 and 2, wherein the monovalent metal salts of alginic acid in the first layer and the second layer are sodium alginate or potassium alginate.

4. The anti-adhesion material according to any one of claims 1-3, wherein the curing agent in the first layer and the second layer is at least one metal ionic compound selected from the group consisting of CaCl₂, CaSO₄, ZnCl₂, SrCl₂, FeCl₃, BaCl₂, CaHPO₄, calcium gluconate, calcium oxalate and calcium lactate.

5. The anti-adhesion material according to any one of claims 1-4, which is configured so that the first layer, if the anti-adhesion material being applied to a wound, faces the surface of the wound.

6. The anti-adhesion material according to any one of claims 1-5, which has one or more of the following characteristics:
(1) when the sponge-like laminate is brought into contact with an agarose gel immersed in phosphate buffered saline for 2-6 hours, the weight increase of the sponge-like laminate is 200-50,000%, when taking the weight of the sponge-like laminate before it is brought into contact with the phosphate buffered saline as a base of 100%;
(2) in a dissolution test that uses elution of a monovalent metal salt of alginic acid via an agarose gel in a phosphate buffer solution at pH 7.5 as an indicator, a proportion of the amount of the monovalent metal salt of alginic acid eluted from the first layer is 30-70% 2 days or later following the start of the measurement, when taking the amount of the monovalent metal salt of alginic acid eluted from the second layer as a base of 100%; and
(3) in a dissolution test that uses elution of a monovalent metal salt of alginic acid via an agarose gel in a phosphate buffer solution at pH 7.5 as an indicator, the monovalent metal salt of alginic acid eluted from the first layer is about 15 ± 5 wt% within 2 days and about 25 ± 10 wt% within 8 days while the monovalent metal salt of alginic acid eluted from the second layer is about 30 ± 8 wt% within 2 days and about 60 ± 10 wt% within 8 days.

7. The anti-adhesion material according to any one of claims 1-6, wherein the sponge-like laminate is pressed.

8. The anti-adhesion material according to any one of claims 1-7, wherein the sponge-like laminate is obtainable by subjecting it to one or more treatments selected from electron-beam sterilization, gamma-ray sterilization, and ethylene oxide gas sterilization.

9. An anti-adhesion material according to any one of claims 1-8 for use in preventing an adhesion of the material to a wound of a subject by applying the first layer of the material to the wound so that it faces the surface of the wound.

10. A method for producing an anti-adhesion material comprising a biocompatible sponge-like laminate according to any one of claims 1 to 9, the method comprising the steps of:
(1) curing a low-endotoxin monovalent metal salt of alginic acid having a weight-average molecular weight of 30,000-300,000 by using a curing agent;
(2) on the monovalent metal salt of alginic acid obtained in (1), curing a low-endotoxin monovalent metal salt of alginic acid having a weight-average molecular weight of 1,000-200,000 by using a curing agent to obtain a laminate; and
(3) lyophilizing the resulting laminate to obtain the sponge-like laminate,
wherein the molecular weights are measured by GPC-MALS method, the sponge-like laminate includes a first sponge-like layer containing the low-endotoxin monovalent metal salt of alginic acid having a weight-average molecular weight of 30,000-300,000 and a second sponge-like layer containing the low-endotoxin monovalent metal salt of alginic acid having a weight-average molecular weight of 1,000-200,000, said low-endotoxin monovalent metal salts of alginic acid of the first and second layer have an endotoxin content of 500 Endotoxin Units (EU)/g or less measured using a limulus reagent, and the weight-average molecular weight of the monovalent metal salt of alginic acid in the first layer is higher than that in the second layer.

11. The method according to claim 10, wherein the curing agent is at least one metal ionic compound selected from the group consisting of CaCl₂, CaSO₄, ZnCl₂, SrCl₂, FeCl₃, BaCl₂, CaHPO₄, calcium gluconate, calcium oxalate and calcium lactate.

12. The method according to either claims 10 and 11, further comprising a step of subjecting the sponge-like laminate obtained in (3) to one or more treatments selected from electron-beam sterilization, gamma-ray sterilization, and ethylene oxide gas sterilization.

13. A biocompatible sponge-like laminate that is porous and is obtainable by the following steps (1)-(3):
(1) curing a low-endotoxin monovalent metal salt of alginic acid having a weight-average molecular weight of 30,000-300,000 by using a curing agent;
(2) on the monovalent metal salt of alginic acid obtained in (1), curing a low-endotoxin monovalent metal salt of alginic acid having a weight-average molecular weight of 1,000-200,000 by using a curing agent to obtain a laminate; and
(3) lyophilizing the resulting laminate to obtain the sponge-like laminate,
wherein the molecular weights are measured by GPC-MALS method, the sponge-like laminate includes a first sponge-like layer containing the low-endotoxin monovalent metal salt of alginic acid having a weight-average molecular weight of 30,000-300,000 and a second sponge-like layer containing the low-endotoxin monovalent metal salt of alginic acid having a weight-average molecular weight of 1,000-200,000, said low-endotoxin monovalent metal salts of alginic acid of the first and second layer have an endotoxin content of 500 Endotoxin Units (EU)/g or less measured using a limulus reagent, and the weight-average molecular weight of the monovalent metal salt of alginic acid in the first layer is higher than that in the second layer, and wherein said sponge-like laminate having one or more of the following characteristics:
(1) the Young's modulus determined based on a stress-strain curve acquired by subjecting a JIS K6251 Type 8-shaped tensile test piece to a tensile tester is 0.3-300 MPa;
(2) the strength at break determined based on the stress-strain curve acquired by subjecting the JIS K6251 Type 8-shaped tensile test piece to the tensile tester is 5-5000 kPa; and
(3) the ratio of the strength at break to the Young's modulus of the sponge-like laminate determined based on a stress-strain curve acquired by subjecting a JIS K6251 Type 8-shaped tensile test piece to a tensile tester is 2-50.

14. The sponge-like laminate according to claim 13, which is used as an anti-adhesion material.

## Patentansprüche

1. Antihaftmaterial, das ein biokompatibles schwammartiges Laminat umfasst, welches porös ist, eine erste und eine zweite schwammartige Schicht, die Alginsäuresalze eines einwertigen Metalls mit geringem Endotoxingehalt enthalten, welche wenigstens teilweise mit einem Härtungsmittel vernetzt sind, umfasst und dadurch erhältlich ist, dass man die gehärtete erste und zweite Schicht des Laminats gemeinsam oder einzeln lyophilisiert, wobei das Gewichtsmittel des Molekulargewichts des Alginsäuresalzes des einwertigen Metalls in der ersten Schicht 30000-300000 beträgt, das Gewichtsmittel des Molekulargewichts des Alginsäuresalzes des einwertigen Metalls in der zweiten Schicht 1000-200000 beträgt, die Alginsäuresalze des einwertigen Metalls mit geringem Endotoxingehalt der ersten und zweiten Schicht einen mit Hilfe eines *Limulus-*Reagens gemessenen Endotoxingehalt von 500 Endotoxineinheiten (EU)/g oder weniger aufweisen, die Gewichtsmittel des Molekulargewichts durch die GPC-MALS-Methode mit anschließender Entnetzungsbehandlung gemessen werden und das Gewichtsmittel des Molekulargewichts des Alginsäuresalzes des einwertigen Metalls in der ersten Schicht höher ist als das Gewichtsmittel des Molekulargewichts des Alginsäuresalzes des einwertigen Metalls in der zweiten Schicht; das Antihaftmaterial eines oder mehrere der folgenden Merkmale aufweist:
(1) der auf der Grundlage einer Spannungs-Dehnungs-Kurve ermittelte Elastizitätsmodul eines Zugprüfkörpers vom Typ JIS K6251 8 beträgt 0,3-300 MPa;
(2) die anhand der Spannungs-Dehnungs-Kurve ermittelte Bruchfestigkeit, die durch Zugversuche mit einem Zugprüfkörper der Form Typ 8 nach JIS K6251 gewonnen wurde, beträgt 5-5000 kPa; und
(3) das Verhältnis der Bruchfestigkeit zum Elastizitätsmodul des schwammartigen Laminats, ermittelt anhand einer Spannungs-Dehnungs-Kurve, die durch Testen eines Zugprüfkörpers der Form Typ 8 nach JIS K6251 mit einer Zugprüfmaschine gewonnen wurde, beträgt 2-50.

2. Antihaftmaterial gemäß Anspruch 1, wobei die Gesamtmenge der Alginsäuresalze eines einwertigen Metalls mit geringem Endotoxingehalt, die in der ersten Schicht und der zweiten Schicht verwendet werden, in einem Bereich von 0,1 mg/cm²-3 mg/cm² liegt.

3. Antihaftmaterial gemäß einem der Ansprüche 1 und 2, wobei es sich bei den Alginsäuresalzen des einwertigen Metalls in der ersten Schicht und in der zweiten Schicht um Natriumalginat oder Kaliumalginat handelt.

4. Antihaftmaterial gemäß einem der Ansprüche 1-3, wobei das Härtungsmittel in der ersten Schicht und in der zweiten Schicht wenigstens eine ionische Metallverbindung ist, die aus der Gruppe ausgewählt ist, welche aus CaCl₂, CaSO₄, ZnCl₂, SrCl₂, FeCl₃, BaCl₂, CaHPO₄, Calciumgluconat, Calciumoxalat und Calciumlactat besteht.

5. Antihaftmaterial gemäß einem der Ansprüche 1-4, das so konfiguriert ist, dass die erste Schicht, wenn das Antihaftmaterial auf eine Wunde aufgetragen wird, der Oberfläche der Wunde zugewandt ist.

6. Antihaftmaterial gemäß einem der Ansprüche 1-5, das eines oder mehrere der folgenden Merkmale aufweist:
(1) wenn das schwammartige Laminat für 2-6 Stunden mit einem in phosphatgepufferter Salzlösung eingetauchten Agarose-Gel in Kontakt gebracht wird, beträgt die Gewichtszunahme des schwammartigen Laminats 200-50000%, wenn das Gewicht des schwammartigen Laminats, bevor es mit der phosphatgepufferten Salzlösung in Kontakt gebracht wird, als Basis von 100% genommen wird;
(2) bei einem Auflösungstest, bei dem die Elution eines Alginsäuresalzes eines einwertigen Metalls über ein Agarose-Gel in einer Phosphatpufferlösung bei pH 7,5 als Indikator verwendet wird, beträgt der Anteil des aus der ersten Schicht eluierten Alginsäuresalzes des einwertigen Metalls zwei Tage nach Beginn der Messung oder später 30-70%, wenn die aus der zweiten Schicht eluierte Menge des Alginsäuresalzes des einwertigen Metalls als Basis von 100% genommen wird; und
(3) bei einem Auflösungstest, bei dem die Elution eines Alginsäuresalzes eines einwertigen Metalls der über ein Agarosegel in einer Phosphatpufferlösung bei pH 7,5 als Indikator verwendet wird, beträgt der Anteil des aus der ersten Schicht eluierten Alginsäuresalzes eines einwertigen Metalls innerhalb von 2 Tagen etwa 15 ± 5 Gew.-% und innerhalb von 8 Tagen etwa 25 ± 10 Gew.-%, während der Anteil des aus der zweiten Schicht eluierten Alginsäuresalzes des einwertigen Metalls innerhalb von 2 Tagen etwa 30 ± 8 Gew.-% und innerhalb von 8 Tagen etwa 60 ± 10 Gew.-% beträgt.

7. Antihaftmaterial gemäß einem der Ansprüche 1-6, wobei das schwammartige Laminat gepresst ist.

8. Antihaftmaterial gemäß einem der Ansprüche 1-7, wobei das schwammartige Laminat ist durch eine oder mehrere Behandlungen, die aus Elektronenstrahlsterilisation, Gammastrahlensterilisation und Ethylenoxidgassterilisation ausgewählt sind, erhältlich.

9. Antihaftmaterial gemäß einem der Ansprüche 1-8 zur Verwendung bei der Verhinderung des Anhaftens des Materials an einer Wunde eines Probanden, indem man die erste Schicht des Materials so auf die Wunde aufträgt, dass es der Oberfläche der Wunde zugewandt ist.

10. Verfahren zur Herstellung eines Antihaftmaterials, das ein biokompatibles schwammartiges Laminat umfasst, gemäß einem der Ansprüche 1 bis 9, wobei das Verfahren die folgenden Schritte umfasst:
(1) Aushärten eines Alginsäuresalzes eines einwertigen Metalls mit geringem Endotoxingehalt und einem Gewichtsmittel des Molekulargewichts von 30000-300000 unter Verwendung eines Härtungsmittels;
(2) Aushärten des Alginsäuresalzes eines einwertigen Metalls mit geringem Endotoxingehalt und mit einem Gewichtsmittel des Molekulargewichts von 1000-200000 mit Hilfe eines Härtungsmittels an dem in (1) erhaltenen Alginsäuresalz des einwertigen Metalls unter Erhalt eines Laminats; und
(3) Lyophilisieren des resultierenden Laminats unter Erhalt des schwammartigen Laminats,
wobei die Molekulargewichte nach der GPC-MALS-Methode gemessen werden, das schwammartige Laminat eine erste schwammartige Schicht, die das Alginsäuresalzes eines einwertigen Metalls mit geringem Endotoxingehalt mit einem Gewichtsmittel des Molekulargewichts von 30000-300000 enthält, und eine zweite schwammartige Schicht, die das Alginsäuresalz eines einwertigen Metalls mit geringem Endotoxingehalt mit einem Gewichtsmittel des Molekulargewichts von 1000-200000 enthält, umfasst, die Alginsäuresalze des einwertigen Metalls mit geringem Endotoxingehalt der ersten und zweiten Schicht einen mit Hilfe eines Limulus-Reagens gemessenen Endotoxingehalt von 500 Endotoxineinheiten (EU)/g oder weniger aufweisen und das Gewichtsmittel des Molekulargewichts des Alginsäuresalzes des einwertigen Metalls in der ersten Schicht höher ist als in der zweiten Schicht.

11. Verfahren gemäß Anspruch 10, wobei das Härtungsmittel wenigstens eine ionische Metallverbindung ist, die aus der Gruppe ausgewählt ist, welche aus CaCl₂, CaSO₄, ZnCl₂, SrCl₂, FeCl₃, BaCl₂, CaHPO₄, Calciumgluconat, Calciumoxalat und Calciumlactat besteht.

12. Verfahren gemäß Anspruch 10 oder 11, weiterhin umfassend einen Schritt des Behandelns des schwammartigen Laminats in einer oder mehreren Behandlungen, die aus Elektronenstrahlsterilisation, Gammastrahlensterilisation und Ethylenoxidgassterilisation ausgewählt sind.

13. Biokompatibles schwammartiges Laminat, das porös ist und durch die folgenden Schritte (1)-(3) erhältlich ist:
(1) Aushärten eines Alginsäuresalzes eines einwertigen Metalls mit geringem Endotoxingehalt und einem Gewichtsmittel des Molekulargewichts von 30000-300000 unter Verwendung eines Härtungsmittels;
(2) Aushärten des Alginsäuresalzes eines einwertigen Metalls mit geringem Endotoxingehalt und mit einem Gewichtsmittel des Molekulargewichts von 1000-200000 mit Hilfe eines Härtungsmittels an dem in (1) erhaltenen Alginsäuresalz des einwertigen Metalls unter Erhalt eines Laminats; und
(3) Lyophilisieren des resultierenden Laminats unter Erhalt des schwammartigen Laminats,
wobei die Molekulargewichte nach der GPC-MALS-Methode gemessen werden, das schwammartige Laminat eine erste schwammartige Schicht, die das Alginsäuresalzes eines einwertigen Metalls mit geringem Endotoxingehalt mit einem Gewichtsmittel des Molekulargewichts von 30000-300000 enthält, und eine zweite schwammartige Schicht, die das Alginsäuresalz eines einwertigen Metalls mit geringem Endotoxingehalt mit einem Gewichtsmittel des Molekulargewichts von 1000-200000 enthält, umfasst, die Alginsäuresalze des einwertigen Metalls mit geringem Endotoxingehalt der ersten und zweiten Schicht einen mit Hilfe eines Limulus-Reagens gemessenen Endotoxingehalt von 500 Endotoxineinheiten (EU)/g oder weniger aufweisen und das Gewichtsmittel des Molekulargewichts des Alginsäuresalzes des einwertigen Metalls in der ersten Schicht höher ist als in der zweiten Schicht und wobei das schwammartige Laminat eines oder mehrere der folgenden Merkmale aufweist:
(1) der auf der Grundlage einer Spannungs-Dehnungs-Kurve ermittelte Elastizitätsmodul eines Zugprüfkörpers vom Typ JIS K6251 8 beträgt 0,3-300 MPa;
(2) die anhand der Spannungs-Dehnungs-Kurve ermittelte Bruchfestigkeit, die durch Zugversuche mit einem Zugprüfkörper der Form Typ 8 nach JIS K6251 gewonnen wurde, beträgt 5-5000 kPa; und
(3) das Verhältnis der Bruchfestigkeit zum Elastizitätsmodul des schwammartigen Laminats, ermittelt anhand einer Spannungs-Dehnungs-Kurve, die durch Testen eines Zugprüfkörpers der Form Typ 8 nach JIS K6251 mit einer Zugprüfmaschine gewonnen wurde, beträgt 2-50.

14. Schwammartiges Laminat gemäß Anspruch 13, das als Antihaftmaterial verwendet wird.

## Revendications

1. Matériau antiadhésif comprenant un stratifié spongieux biocompatible qui est poreux, qui comporte une première et une deuxième couches spongieuses contenant des sels métalliques monovalents d'acide alginique à faible teneur en endotoxines qui sont réticulés au moins partiellement avec un agent de durcissement, et qui peut être obtenu en lyophilisant conjointement ou séparément les première et deuxième couches durcies du stratifié, dans lequel le poids moléculaire moyen en poids du sel métallique monovalent d'acide alginique dans la première couche est de 30 000-300 000, le poids moléculaire moyen en poids du sel métallique monovalent d'acide alginique dans la deuxième couche est de 1000-200 000, lesdits sels métalliques monovalents d'acide alginique à faible teneur en endotoxines de la première et de la deuxième couche ont une teneur en endotoxines de 500 Unités d'Endotoxine (EU)/g ou moins, mesurée à l'aide d'un réactif Limulus, les poids moléculaires moyens en poids sont mesurés par la méthode GPC-MALS (Chromatographie par perméation de gel - Diffusion de la lumière multi-angle) après un traitement de déréticulation, et le poids moléculaire moyen en poids du sel métallique monovalent d'acide alginique dans la première couche est supérieur au poids moléculaire moyen en poids du sel métallique monovalent d'acide alginique dans la deuxième couche, ledit matériau antiadhésif ayant une ou plusieurs des caractéristiques suivantes :
(1) le module d'Young, déterminé à partir d'une courbe contrainte-déformation obtenue en soumettant une éprouvette d'essai de traction en forme de 8, type JIS K6251, à un appareil d'essai de traction, est de 0,3-300 MPa ;
(2) la résistance à la rupture, déterminée à partir de la courbe contrainte-déformation obtenue en soumettant l'éprouvette d'essai de traction en forme de 8, type JIS K6251, à l'appareil d'essai de traction, est de 5-5000 kPa ; et
(3) le rapport de la résistance à la rupture au module d'Young du stratifié spongieux, déterminé à partir d'une courbe contrainte-déformation obtenue en soumettant une éprouvette d'essai de traction en forme de 8, type JIS K6251, à un appareil d'essai de traction, est de 2-50.

2. Matériau antiadhésif selon la revendication 1, dans lequel la quantité totale des sels métalliques monovalents d'acide alginique à faible teneur en endotoxines utilisée dans la première couche et dans la deuxième couche est comprise dans un intervalle de 0,1 mg/cm²-3 mg/cm².

3. Matériau antiadhésif selon l'une ou l'autre des revendications 1 et 2, dans lequel les sels métalliques monovalents d'acide alginique dans la première couche et dans la deuxième couche sont de l'alginate de sodium ou de l'alginate de potassium.

4. Matériau antiadhésif selon l'une quelconque des revendications 1-3, dans lequel l'agent de durcissement dans la première couche et dans la deuxième couche est au moins un composé ionique métallique choisi dans le groupe constitué par CaCl₂, CaSO₄, ZnCl₂, SrCl₂, FeCl₃, BaCl₂,CaHPO₄, le gluconate de calcium, l'oxalate de calcium et le lactate de calcium.

5. Matériau antiadhésif selon l'une quelconque des revendications 1-4, qui est configuré de manière à ce que, si le matériau antiadhésif est appliqué sur une plaie, la première couche soit en contact avec la surface de la plaie.

6. Matériau antiadhésif selon l'une quelconque des revendications 1-5, qui possède une ou plusieurs des caractéristiques suivantes :
(1) lorsque le stratifié spongieux est mis en contact avec un gel d'agarose immergé dans une solution saline tamponnée au phosphate pendant 2-6 heures, l'augmentation du poids du stratifié spongieux est de 200-50 000%, en prenant le poids du stratifié spongieux avant qu'il n'entre en contact avec la solution saline tamponnée au phosphate comme base de 100% ;
(2) dans un test de dissolution qui utilise l'élution d'un sel métallique monovalent d'acide alginique via un gel d'agarose dans une solution tampon phosphate à pH 7,5 comme indicateur, la proportion de la quantité du sel métallique monovalent d'acide alginique élué à partir de la première couche est de 30-70% 2 jours ou plus après le début de la mesure, en prenant la quantité de sel métallique monovalent d'acide alginique élué à partir de la deuxième couche comme base de 100% ; et
(3) dans un test de dissolution qui utilise l'élution d'un sel métallique monovalent d'acide alginique via un gel d'agarose dans une solution tampon phosphate à pH 7,5 comme indicateur, le sel métallique monovalent d'acide alginique élué à partir de la première couche est d'environ 15 ± 5% en poids en 2 jours et environ 25 ± 10% en poids en 8 jours tandis que le sel métallique monovalent d'acide alginique élué à partir de la deuxième couche est d'environ 30 ± 8% en poids en 2 jours et environ 60 ± 10% en poids en 8 jours.

7. Matériau antiadhésif selon l'une quelconque des revendications 1-6, dans lequel le stratifié spongieux est pressé.

8. Matériau antiadhésif selon l'une quelconque des revendications 1-7, dans lequel le stratifié spongieux peut être obtenu en le soumettant à un ou plusieurs traitements choisis parmi la stérilisation par faisceau d'électrons, la stérilisation par rayons gamma et la stérilisation à l'oxyde d'éthylène.

9. Matériau antiadhésif selon l'une quelconque des revendications 1-8 destiné à empêcher l'adhérence du matériau à une plaie d'un sujet en appliquant la première couche du matériau sur la plaie de manière à ce qu'elle soit en contact avec la surface de la plaie.

10. Procédé de production d'un matériau antiadhésif comprenant un stratifié spongieux biocompatible selon l'une quelconque des revendications 1 à 9, ledit procédé comprenant les étapes consistant à :
(1) durcir un sel métallique monovalent d'acide alginique à faible teneur en endotoxines ayant un poids moléculaire moyen en poids de 30 000-300 000 en utilisant un agent de durcissement ;
(2) sur le sel métallique monovalent d'acide alginique obtenu dans l'étape (1), durcir un sel métallique monovalent d'acide alginique à faible teneur en endotoxines ayant un poids moléculaire moyen en poids de 1000-200 000 en utilisant un agent de durcissement pour obtenir un stratifié ; et
(3) lyophiliser le stratifié résultant pour obtenir le stratifié spongieux,
dans lequel les poids moléculaires sont mesurés par la méthode GPC-MALS, le stratifié spongieux comporte une première couche spongieuse contenant le sel métallique monovalent d'acide alginique à faible teneur en endotoxines ayant un poids moléculaire moyen en poids de 30 000-300 000 et une deuxième couche spongieuse contenant le sel métallique monovalent d'acide alginique à faible teneur en endotoxines ayant un poids moléculaire moyen en poids de 1000-200 000, lesdits sels métalliques monovalents d'acide alginique à faible teneur en endotoxines de la première et de la deuxième couches ont une teneur en endotoxine de 500 Unités d'Endotoxine (EU)/g ou moins, mesurée à l'aide d'un réactif Limulus, et le poids moléculaire moyen en poids du sel métallique monovalent d'acide alginique dans la première couche est supérieur à celui dans la deuxième couche.

11. Procédé selon la revendication 10, dans lequel l'agent de durcissement est au moins un composé ionique métallique choisi dans le groupe constitué par CaCl₂, CaSO₄, ZnCl₂, SrCl₂, FeCl₃, BaCl₂,CaHPO₄, le gluconate de calcium, l'oxalate de calcium et le lactate de calcium.

12. Procédé selon l'un ou l'autre des revendications 10 et 11, comprenant en outre une étape consistant à soumettre le stratifié spongieux obtenu dans l'étape (3) à un ou plusieurs traitements choisis parmi la stérilisation par faisceau d'électrons, la stérilisation par rayons gamma et la stérilisation à l'oxyde d'éthylène.

13. Stratifié spongieux biocompatible qui est poreux et pouvant être obtenu par les étapes (1)-(3) suivantes qui consistent à :
(1) durcir un sel métallique monovalent d'acide alginique à faible teneur en endotoxines ayant un poids moléculaire moyen en poids de 30 000-300 000 en utilisant un agent de durcissement ;
(2) sur le sel métallique monovalent d'acide alginique obtenu dans l'étape (1), durcir un sel métallique monovalent d'acide alginique à faible teneur en endotoxines ayant un poids moléculaire moyen en poids de 1000-200 000 en utilisant un agent de durcissement pour obtenir un stratifié ; et
(3) lyophiliser le stratifié résultant pour obtenir le stratifié spongieux,
où les poids moléculaires sont mesurés par la méthode GPC-MALS, le stratifié spongieux comporte une première couche spongieuse contenant le sel métallique monovalent d'acide alginique à faible teneur en endotoxines ayant un poids moléculaire moyen en poids de 30 000-300 000 et une deuxième couche spongieuse contenant le sel métallique monovalent d'acide alginique à faible teneur en endotoxines ayant un poids moléculaire moyen en poids de 1000-200 000, lesdits sels métalliques monovalents d'acide alginique à faible teneur en endotoxines de la première et de la deuxième couches ont une teneur en endotoxine de 500 Unités d'Endotoxine (EU)/g ou moins, mesurée à l'aide d'un réactif Limulus, et le poids moléculaire moyen en poids du sel métallique monovalent d'acide alginique dans la première couche est supérieur à celui dans la deuxième couche, ledit stratifié spongieux ayant une ou plusieurs des caractéristiques suivantes :
(1) le module d'Young, déterminé à partir d'une courbe contrainte-déformation obtenue en soumettant une éprouvette d'essai de traction en forme de 8, type JIS K6251, à un appareil d'essai de traction, est de 0,3-300 MPa ;
(2) la résistance à la rupture, déterminée à partir de la courbe contrainte-déformation obtenue en soumettant l'éprouvette d'essai de traction en forme de 8, type JIS K6251, à l'appareil d'essai de traction, est de 5-5000 kPa ; et
(3) le rapport de la résistance à la rupture au module d'Young du stratifié spongieux, déterminé à partir d'une courbe contrainte-déformation obtenue en soumettant une éprouvette d'essai de traction en forme de 8, type JIS K6251, à un appareil d'essai de traction, est de 2-50.

14. Stratifié spongieux selon la revendication 13, qui est utilisé comme matériau antiadhésif.
